# EUROPEAN PATENT SPECIFICATION

(11) **EP 2 992 865 B1**
(45) Date of publication and mention of the grant of the patent: **06.11.2019**
(21) Application number: 14183778.1
(22) Date of filing: 05.09.2014
(51) Int. Cl.: A61F 13/49, A61F 13/56, A61F 13/15

(54) **Absorbent article with aysmmetrical side panels**
Absorbierender Artikel mit asymmetrischen Seitenwänden
Article absorbant avec panneaux latéraux asymétriques

(43) Date of publication of application: 09.03.2016
(73) Proprietor: Drylock Technologies N.V., 9240 Zele (BE)
(72) Inventor: Van Ingelgem, Werner, 9240 Zele (BE)
(74) Representative: D'Halleweyn, Nele Veerle Trees Gertrudis

(56) References cited:
- EP-A1- 0 808 145
- EP-A1- 2 410 964
- US-A1- 2011 088 233
- US-A1- 2014 163 500

## Description

### TECHNICAL FIELD

The invention pertains to the technical field of absorbent articles comprising side panels, such as incontinence diapers, pants and the like, and provides a method to produce said side panels.

### BACKGROUND

Absorbent articles such as diapers, pants and the like, typically contain a topsheet, backsheet and an absorbent article in between the topsheet and backsheet to form a chassis of the absorbent article. Typically side panels are attached to the front and/or the back of the chassis to provide the absorbent article with extensions in the front and/or the back that can encircle the waist of a wearer during use.

Several configurations of such side panels are known in the art, as for example provided in EP 2 410 964 or WO 2005 102 241 and US 2011/0088233 A1, which relates to a method of providing a disposable product with a contoured leg opening.

The shape of the side panels is essential to allow an optimal fit around the waist of a wearer during use. Especially when dealing with diapers and pants intended to be used by adults, the shape of the side panels becomes very important as these side panels typically display much larger dimensions and have to encompass a much larger waist circumference than in the case of, for example, infants or babies. Moreover, in a world where obesity remains a growing problem, there is an increased demand for proper fitting diapers or pants for obese people suffering from incontinence.

Besides comfort, it is also important from an environmental and economic viewpoint that the side panels can be made in a facile manner with limited loss of material when making the side panels. However, to obtain side panels with a specific shape, most often material is lost during production, which is discarded as waste.

There remains a need in the art for improved side panels that provide an optimal balance between a comfortable fit around the waist of a wearer and a facile fabrication of those side panels, preferably with limiting loss of material while making them.

The present invention aims to resolve at least some of the problems mentioned above.

The invention thereto aims to provide an absorbent article of which the side panels are provided with an improved shape that allow an optimized fit around the waist of a wearer, which can be manufactured in a facile, cost effective manner.

### SUMMARY OF THE INVENTION

The present invention provides a disposable absorbent incontinence article according to claim 1.

An absorbent article according to the present invention contains at least one side panel with an optimized shape which allows an improved fit of the absorbent article around the waist of a wearer, especially when the wearer is an adult with a large circumference, while still allowing it to be manufactured in a facile, cost effective manner. In a preferred embodiment of the current invention, the side panels are made from the same web material without loss of web material when making the side panels.

In a second aspect, the present invention provides a side panel according to claim 12 preferably a side panel for use in an absorbent article according to claim 1.

In a final aspect, the present invention provides a method for producing a side panel according to claim 14 preferably a side panel according to claim 12. This method allows side panels to be made with minimal to no loss of web material during production.

### DESCRIPTION OF FIGURES

**Figure 1** provides a schematic view of an absorbent article according to a preferred embodiment of the current invention, displaying a garment-facing side of an adult incontinence diaper in a flat out, un-contracted state.
**Figure 2** provides a closer schematic view of the rear side panel of the absorbent according to Figure 2.
**Figure 3** provides a schematic view of an example of a side panel according to the prior art.
**Figure 4** provides a schematic view of another example of a side panel according to the prior art.
**Figure 5** displays a schematic representation of a method to produce side panels according to an embodiment of the current invention.
**Figure 6** displays a schematic representation of a method to produce side panels according to another embodiment of the current invention.
**Figure 7** displays a schematic representation of a method to produce side panels according to another embodiment of the current invention.
**Figure 8** displays a schematic representation of a method to produce side panels according to other embodiments of the current invention and displays a few of the potential shapes the side panels can adapt.

### DETAILED DESCRIPTION OF THE INVENTION

The present invention concerns a disposable absorbent incontinence article which is provided at least one side panels that displays an improved fit around the waist of a wearer and provides a method to allow the side panel to be produced in a facile, cost effective manner.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a layer" refers to one or more than one layer.
"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, insofar such variations are appropriate to perform in the disclosed invention. However, it is to be understood that the value to which the modifier "about" refers is itself also specifically disclosed.
"Absorbent article", "absorbent garment", "absorbent product", "absorbing article", "absorbing garment", "absorbing product" and the like as used herein are used interchangeably and refer to devices that absorb and contain bodily exudates, and more specifically, refers to devices that are placed against or in proximity to the body of the wearer to absorb and contain the various liquids discharged from the body. Absorbent articles include but are not limited to feminine hygiene garments, baby diapers and pants, adult incontinence garments, various diaper and pants holders, liners, towels, absorbent inserts and the like.
"Absorbent core" as used herein refers to a three-dimensional part of the absorbent structure, comprising liquid-absorbing material, useful to absorb and/or retain bodily exudates.
"Absorbent component" as used herein refers to a structural constituent of an absorbent structure, e.g., a piece of an absorbent core, such as one of multiple pieces in a multi-piece absorbent core.
"Absorbent element" as used herein refers to a part of a functional constituent of an absorbent structure, e.g., a liquid acquisition layer, a liquid distribution layer, or a liquid storage layer formed of a material or materials having particular liquid handling characteristics suitable for the specific function.
"Absorbent insert" as used herein refers to a device adapted for insertion into an absorbent article and to serve as an absorbent structure when so inserted.
"Absorbent layer" as used herein refers to a term referring to a discrete, identifiable sheet-like or web-like element of an absorbent structure which may remain detached and relatively movable with respect to another such element or may be attached or joined so as to remain permanently associated with another such element. Each absorbent layer may itself include a laminate or combination of several layers, sheets and/or webs of similar or diverse compositions.
"Absorbent polymer material", "absorbent gelling material", "AGM", "superabsorbent", "superabsorbent material", "super absorbent polymer", "SAP" and the like as used herein are used interchangeably and refer to any suitable particulate (e.g., flaked, particulate, granular, or powdered) or fibrous cross linked polymeric materials that can absorb at least 5 times and preferably at least about 10 times or more its weight of an aqueous 0.9% saline solution as measured using the Centrifuge Retention Capacity test (EDANA 441.2-01).
"Absorbent polymer material area" as used herein refers to the area of the absorbent structure wherein adjacent layers are separated by a multiplicity of absorbent polymer material. Incidental contact areas between these adjacent layers within the absorbent particulate polymer material area may be intentional (e.g bond area's) or unintentional (e.g. manufacturing artefacts).
"Absorbent particulate polymer material" as used herein refers to an absorbent polymer material which is in particulate form such as powders, granules, flakes and the like so as to be flowable in the dry state.
"Absorption" as used herein refers to the process by which a liquid is taken up within a material.
"Acquisition layer", "acquisition region", "acquisition surface" or "acquisition material" and the like as used herein refer to a layer having a faster liquid uptake capability.
"Absorbency" is the ability of a material to take up fluids by various means including capillary, osmotic, solvent, chemical or other action.
"Adult incontinence garment" as used herein refers to absorbent articles intended to be worn by incontinent adults, for absorbing and containing bodily exudates.
"Adhesion" as used herein refers to the force that holds different materials together at their interface.
"Adhesive" as used herein refers to a material, which may or may not be flowable in solution or when heated, that is used to bond materials together.
"Adsorption" as used herein refers to the process by which a liquid is taken up by the surface of a material.
"Airlaying" as used herein refers to forming a web by dispersing fibres or particles in an air stream and condensing them from the air stream onto a moving screen by means of a pressure or vacuum; a web of fibres produced by airlaying is herein referred to an "airlaid"; an airlaid web bonded by one or more techniques to provide fabric integrity is herein referred to an "airlaid nonwoven".
"Apparent density", "density" as used herein refers to the basis weight of the sample divided by the calliper with appropriate unit conversions incorporated therein. Apparent density used herein has the unit g/cm3.
"Attach", "attached" and "attachment" as used herein are synonymous with their counterparts of the terms "fasten", "affix", "secure", "glue", "bind", "join" and "link".
"Baby diaper" as used herein refers to absorbent articles intended to be worn by children, for absorbing and containing bodily exudates which the user draws up between the legs and fastens about the waist of the wearer.
"Baby pants" as used herein refers to absorbent articles marketed for use in transitioning children from diapers to underwear intended to cover the lower torso of children, so as to absorb and contain body exudates which article is generally configured like a panty garment and manufactured with a completed waist encircling portion, thereby eliminating the need for the user to fasten the article about the waist of the wearer.
"Rear region" as used herein refers to the portion of an absorbent article or part thereof that is intended to be positioned proximate the back or rear of a wearer.
"Backing" as used herein refers to a web or other material that supports and reinforces the back of a product.
"Basis weight" is the weight per unit area of a sample reported in grams per square meter, g/m2 or gsm.
"Bodily exudates", "body exudates", "bodily fluids", "body fluids", "bodily discharges", "body discharges", "liquids" and the like as used herein are used interchangeably and refer to, but are not limited to urine, blood, vaginal discharges, breast milk, sweats and faecal matter.
"Binder", "adhesive", "glue", "resins", "plastics" and the like as used herein are used interchangeably and refer to substances, generally in a solid form (e.g. powder, film, fibre) or as a foam, or in a liquid form (e.g. emulsion, dispersion, solution) used for example by way of impregnation, spraying, printing, foam application and the like used for attaching or bonding functional and/or structural components, elements and materials, for example including heat and/or pressure sensitive adhesives, hot-melts, heat activated adhesives, thermoplastic materials, chemical activated adhesives/solvents, curable materials and the like.
"Bond strength" as used herein refers to the amount of adhesion between bonded surfaces. It is a measure of the stress required to separate a layer of material from the base to which it is bonded.
"Capillary action", "capillarity", or "capillary motion" and the like as used herein are used to refer to the phenomena of the flow of liquid through porous media.
"Chassis" as used herein refers to a foundational constituent of an absorbent article upon which the remainder of the structure of the article is built up or overlaid, e.g., in a diaper, the structural elements that give the diaper the form of briefs or pants when configured for wearing, such as a backsheet, a topsheet, or a combination of a topsheet and a backsheet.
"Cellulose fibres" as used herein refers to naturally occurring fibres based on cellulose, such as, for example cotton, linen, etc; wood pulp fibres are one example of cellulose fibres; man-made fibres derived from cellulose, such as regenerated cellulose (rayon), or partially or fully acetylated cellulose derivatives (e.g. cellulose acetate or triacetate) are also considered as cellulose fibres.
"Cluster" or the like as used herein refers to an agglomeration of particles and/or fibres.
"Chemically stiffened fibres", chemically modified fibres", "chemically cross-linked fibres", "curly fibres" and the like as used herein are used interchangeably and refer to any fibres which have been stiffened by chemical means to increase stiffness of the fibres under both dry and aqueous conditions, for example by way of addition of chemical stiffening agents (e.g. by coating, impregnating, etc), altering the chemical structure of the fibres themselves (e.g. by cross-linking polymer chains, etc) and the like.
"Cohesion" as used herein refers to the resistance of similar materials to be separated from each other.
"Compartment" as used herein refers to chambers, cavities, pockets and the like.
"Comprise," "comprising," and "comprises" and "comprised of" as used herein are synonymous with "include", "including", "includes" or "contain", "containing", "contains" and are inclusive or open-ended terms that specify the presence of what follows e.g. a component and do not exclude or preclude the presence of additional, non-recited components, features, elements, members, steps, known in the art or disclosed therein.
"Coverstock" as used herein refers to a lightweight non-woven material used to contain and conceal an underlying absorbent core material; examples are the facing layer or materials that cover the absorbent cores of feminine hygiene garments, baby diapers and pants and adult incontinence garments.
"Crotch region" of an absorbent article as used herein refers to about 50% of the absorbent article's total length (i.e., in the y-dimension), where the crotch point is located in the longitudinal centre of the crotch region. That is, the crotch region is determined by first locating the crotch point of the absorbent article, and then measuring forward and backward a distance of 25% of the absorbent article's total length.
"Cross direction (CD)", "lateral" or "transverse" and the like as used herein are used interchangeably and refer to a direction which is orthogonal to the longitudinal direction and includes directions within ±45° of the transversal direction.
"Curing" as used herein refers to a process by which resins, binders or plastics are set into or onto fabrics, usually by heating, to cause them to stay in place; the setting may occur by removing solvent or by cross-linking so as to make them insoluble.
"Diaper", "conventional diaper", "diaper-like", "diaper-like garment" and the like as used herein are used interchangeably and refer to disposable absorbent articles, which typically include a front waist portion and a rear waist portion which may be releasably connected about the hips of the wearer during use by conventional fasteners such as adhesive tape fasteners or hook and loop type fasteners. In use, the article is positioned between the legs of the wearer and the fasteners are releasably attached to secure the rear waist portion to the front waist portion of the diaper, thereby securing the diaper about the waist of the wearer. The front waist portion and a rear waist portion are connected by relatively non-stretchable or stretchable members (the term "stretchable" as used herein refers to materials that are extensible when forces are applied to the material, and offer some resistance to extension). Hence, such articles are generally not configured to be pulled up or down over the hips of the wearer when the fasteners are attached.
"Disposable" is used herein to describe articles that are generally not intended to be laundered or otherwise restored or reused (i.e., they are intended to be discarded after a single use and, preferably, to be recycled, composted or otherwise disposed of in an environmentally compatible manner).
"Distribution layer", "distribution region", "distribution surface" or "distribution material" and the like as used herein are used interchangeably and refer to a layer having a larger capacity in wicking, dispersing and distributing liquids.
"Drylaying" as used herein refers to a process for making a nonwoven web from dry fibre; these terms apply to the formation of carded webs, as well as to the air laying formation of random webs; a web of fibres produced by drylaying is herein referred to as a "drylaid"; a drylaid web bonded by one or more techniques to provide fabric integrity is herein referred to a "drylaid nonwoven".
"Dry strength" as used herein refers to the strength of an adhesive joint determined in dry state conditions, immediately after drying under specified conditions or after a period of conditioning in the standard laboratory atmosphere.
"Fabric" as used herein refers to a sheet structure made from fibres, filaments and/or yarns.
"Feminine hygiene garments" as used herein refer to absorbent hygiene articles intended to be worn by woman, for absorbing and containing body exudates.
"Fibre" as used herein refers to the basic threadlike structure from which nonwovens, yarns and textiles are made. It differs from a particle by having a length at least 4 times its width; "Natural fibres" are either of animal (wool, silk), vegetable (cotton, flax, jute) or mineral (asbestos) origin, while "Man-made fibres" may be either polymers synthesised from chemical compounds (polyester, polypropylene, nylon, acrylic etc.) or modified natural polymers (rayon, acetate) or mineral (glass). "Fibre" and "filament" are used interchangeably.
"Fluff pulp" as used herein refers to wood pulp specially prepared to be drylaid.
"Front region" as used herein refers to the portion of an absorbent article or part thereof that is intended to be positioned proximate the front of a wearer.
"Garment facing layer" as used herein refers to elements of the chassis that form the outer surface of the absorbent article, such as the backsheet, the side panels, the waist fasteners, and the like, when such elements are present.
"Heat activated adhesive" as used herein refers to a dry adhesive that is rendered tacky or fluid by application of heat or heat and pressure to the assembly.
"Heat sealing adhesive" as used herein refers to a thermoplastic adhesive which is melted between the adherent surfaces by heat application to one or both of the adjacent adherent surfaces.
"Highloft" as used herein refers to general term of low density, thick or bulky fabrics.
"Hot-melt adhesive" as used herein refers to a solid material that melts quickly upon heating, then sets to a firm bond upon cooling; used for almost instantaneous bonding.
"Hydrophilic" as used herein refers to having an affinity for being wetted by water or for absorbing water.
"Hydrophobic" as used herein refers to lacking the affinity for being wetted by water or for absorbing water.
"Immobilisation layer" as used herein refers to a layer able to be applied to the absorbent polymer material or absorbent polymer material area with the intent to bond and/or immobilize absorbent material and/or absorbent layer.
"Join", "joined" and "joining" as used herein refers to encompassing configurations wherein an element is directly secured to another element by affixing the element directly to the other element, as well as configurations wherein the element is indirectly secured to the other element by affixing the element to an intermediate member or members which in turn is or are affixed to the other element.
"Knitting" as used herein refers to the technique for interlocking loops of fibres with needles or similar devices.
"Layer" refers to identifiable components of the absorbent article, and any part referred to as a "layer" may actually comprise a laminate or combination of several sheets or webs of the requisite type of materials. As used herein, the term "layer" includes the terms "layers" and "layered." "Upper" refers to the layer of the absorbent article which is nearest to and faces the wearer facing layer; conversely, the term "lower" refers to the layer of the absorbent article which is nearest to and faces the garment facing layer. "Layer" is three dimensional structure with a x dimension width, y dimension length, and z-dimensions thickness or calliper, said x-y dimensions being substantially in the plane of the article, however it should be noted that the various members, layers, and structures of absorbent articles according to the present invention may or may not be generally planar in nature, and may be shaped or profiled in any desired configuration.
"Machine direction (MD)", "longitudinal" and the like as used herein are used interchangeably and refer to a direction running parallel to the maximum linear dimension of the structure and includes directions within ±45° of the longitudinal direction.
"Major surface" as used herein refers to a term used to describe the surfaces of greatest extent of a generally planar or sheet-like structural element and to distinguish these surfaces from the minor surfaces of the end edges and the side edges, i.e., in an element having a length, a width, and a thickness, the thickness being the smallest of the three dimensions, the major surfaces are those defined by the length and the width and thus having the greatest extent.
"Mass flow" as used herein refers to the flow of a liquid from one absorbent element or component to another absorbent element or component by channel flow action.
"Mechanical bonding" as used herein refers to a method of bonding fibres by entangling them. This can be achieved by needling, stitching with fibres or by the use of high-pressure air or water jets and the like.
"Non-woven" as used herein refers to manufactured sheet, web or batt of directionally or randomly orientated fibres, bonded by friction, and/or cohesion and/or adhesion, excluding paper and products which are woven, knitted, tufted, stitch-bonded incorporating binding yarns or filaments, or felted by wet-milling, whether or not additionally needled. The fibres may be of natural or man-made origin and may be staple or continuous filaments or be formed in situ. Commercially available fibres have diameters ranging from less than about 0.001 mm to more than about 0.2 mm and they come in several different forms: short fibres (known as staple, or chopped), continuous single fibres (filaments or monofilaments), untwisted bundles of continuous filaments (tow), and twisted bundles of continuous filaments (yarn). Nonwoven fabrics can be formed by many processes such as melt blowing, spun bonding, solvent spinning, electrospinning, and carding. The basis weight of nonwoven fabrics is usually expressed in grams per square meter (gsm).
"Pant", "training pant", "closed diapers", "prefastened diapers", "pull-on diapers" and "diaper-pants" and the like as used herein are used interchangeably and refer to absorbent articles which are typically applied to the wearer by first leading the feet into the respective leg openings and subsequently pulling the pants from the feet to waist area over the hips and buttocks of the wearer and which are capable of being pulled up or down over the hips of the wearer. Typically, such articles may include a front waist portion and a rear waist portion which may be connected about the hips of the wearer by integral or releasable members. A pant may be preformed by any suitable technique including, but not limited to, joining together portions of the article using refastenable and/or non-refastenable bonds (e.g., seam, weld, adhesive, cohesive bond, fastener, etc.). A pant may be preformed anywhere along the circumference of the article (e.g., side fastened, front waist fastened).
"Polymer" as used herein refers to but is not limited to, homopolymers, copolymers, such as for example, block, graft, random and alternating copolymers, terpolymers, etc. and blends and modifications thereof. Unless otherwise specifically limited, the term "polymer" includes all possible spatial configurations of the molecule and include, but are not limited to isotactic, syndiotactic and random symmetries.
"Rear" as used herein refers to the portion of an absorbent article or part thereof that is intended to be positioned proximate the back or rear of the wearer.
"Resin" as used herein refers to a solid or semisolid polymeric material.
"Substantially cellulose free" as used herein refers to an absorbent article, structure or core, that contains less than 20% by weight cellulosic fibres, less than 10% cellulosic fibres, less than 5% cellulosic fibres, no cellulosic fibres, or no more than an immaterial amount of cellulosic fibres which do not materially affect the thinness, flexibility or absorbency thereof.
"Thermobonding" as used herein refers to a method of bonding fibres by the use of heat and/or high-pressure.
"Thermoplastic" as used herein refers to polymeric materials that have a melting temperature and can flow or be formed into desired shapes on the application of heat at or below the melting point.
"Ultrasonic" as used herein refers to the use of high frequency sound to generate localised heat through vibration thereby causing thermoplastic fibres to bond to one another.
"Water-absorbing", "liquid-absorbing", "absorbent", "absorbing" and the like as used herein are used interchangeably and refer to compounds, materials, products that absorb at least water, but typically also other aqueous fluids and typically other parts of bodily exudates such as at least urine or blood.
"Wearer facing layer" as used herein refers to elements of the chassis that form the inner surface of the absorbent article, such as the topsheet, the leg cuffs, and the side panels, etc., when such elements are present.
"Weaving" as used herein refers to the process of interlacing two or more sets of yarns at right angles to form a fabric; a web of fibres produced by weaving is herein referred to as a "Woven".
"Web material" as used herein refers to an essentially endless material in one direction, i.e. the longitudinal extension or the length, or the x- direction in Cartesian coordinates relative to the web material. Included in this term is an essentially unlimited sequence of pieces cut or otherwise separated from an essentially endless material. Often, though not necessarily, the web materials will have a thickness dimension (i.e. the z-direction) which is significantly smaller than the longitudinal extension (i.e. in x-direction). Typically, the width of web materials (the y-direction) will be significantly larger than the thickness, but less than the length. Often, though not necessarily, the thickness and the width of such materials is essentially constant along the length of the web. Without intending any limitation, such web materials may be cellulosic fibre materials, tissues, woven or non-woven materials and the like. Typically, though not necessarily, web materials are supplied in roll form, or on spools, or in a folded state in boxes. The individual deliveries may then be spliced together to form the essentially endless structure. A web material may be composed of several web materials, such as multilayer non-woven, coated tissues, non woven / film laminates. Web materials may comprise other materials, such as added binding material, particles, hydrophilizing agents and the like.
"Wet burst strength" is a measure of a layer's ability to absorb energy, when wet and subjected to deformation normal to the plane of the web.
"Wet strength" as used herein refers to the strength of a joint determined immediately after removal from a liquid in which it has been immersed under specified conditions of time, temperature and pressure. The term is commonly used in the art to designate strength after immersion in water.
"Wetlaying" as used herein refers to the forming a web from an aqueous dispersion of fibres by applying modified paper making techniques; a web of fibres produced by wetlaying is herein referred to as a "wetlaid".
"Wood pulp" as used herein refers to cellulosic fibres used to make viscose rayon, paper and the absorbent cores of products such as feminine hygiene garments, baby diapers and pants and adult incontinence garments.
"X-y dimension" as used herein refers to the plane orthogonal to the thickness of the article, structure or element. The x- and y-dimensions correspond generally to the length and width, respectively, of the article, structure or element.
"Z-dimension" as used herein refers to the dimension orthogonal to the length and width of the article, structure or element. The z-dimension corresponds generally to the thickness of the article, structure or element.

The recitation of numerical ranges by endpoints includes all numbers and fractions subsumed within that range, as well as the recited endpoints.

In a first aspect, the invention provides a disposable absorbent incontinence article comprising a liquid permeable topsheet, a liquid impermeable backsheet and an absorbent core positioned in between the topsheet and the backsheet, whereby the topsheet, backsheet and absorbent core form a chassis of the absorbent article. The absorbent article further comprises a front region, a rear region and a crotch region, which crotch region interconnects the front and the rear region, and a longitudinal axis which extends from the front region over the crotch region to the rear region, as well as a transverse axis which is generally perpendicular to the longitudinal axis. The chassis comprises two transversely opposed side edges substantially extending along the longitudinal axis, and a front edge and a rear edge at respectively the front region and rear region, which front edge and rear edge substantially extend along the transverse axis.

The absorbent article according to the present invention comprises at least one front side panel attached to the front region and/or at least one rear side panel attached to the rear region. In a preferred embodiment, the absorbent article comprises at least one front side panel attached to the front region and at least one rear side panel attached to the rear region. In a more preferred embodiment, the absorbent article comprises two front side panels attached to the front region, one on each transversely opposed side edge thereof, and/or two rear side panels attached to the rear region, one on each transversely opposed side edge thereof. In a most preferred embodiment, the absorbent article comprises two front side panels attached to the front region, one on each transversely opposed side edge thereof, and two rear side panels attached to the rear region, one on each transversely opposed side edge thereof.

The front and/or rear side panel comprises a proximal edge and a distal edge which are transversely opposed, and an upper edge and a lower edge, each extending between the proximal to the distal edge, the upper edge being located closer to the front edge or rear edge of the chassis than the lower edge for respectively a front side panel and a rear side panel. The side panels are preferably attached to the chassis of the absorbent article in such a manner that they extend in a transverse direction beyond the side edges of the chassis. The proximal edge is hereby preferably the edge closest to the side edge of the chassis from which the side panel extends and which distal edge is spaced farthest away from said side edge.

In particular, the proximal and the distal edge of the front and/or rear side panel according to the present invention substantially run parallel to each other along the longitudinal axis, whereby the upper edge of the front and/or the rear side panel makes a non-zero angle alpha with the transverse axis. "Alpha" or the symbol "α" are used herein as synonyms. The angle alpha preferably has a value between 0.5 and 30 °, more preferably between 1 and 20 °, even more preferably between 1 and 10 °, even more preferably between 2 and 9 °, most preferably between 4 and 8 °. In a preferred embodiment the angle alpha has a value of about 1 °, 2 °, 3 °, 4 °, 5 °, 6 °, 7 °, 8 °, 9 °, 10 °, 11 °, 12 °, 13 °, 14 °, 15 °, 16 °, 17 °, 18 °, 19 °, 20 °, 21 °, 22 °, 23 °, 24 °, 25 °, 26 °, 27 °, 28 °, 29 ° or 30 °.

In one embodiment, the absorbent article comprises at least one front side panel, preferably two front side panels, of which the upper edge makes a non-zero angle alpha with the transverse axis, whereby the non-zero angle alpha preferably has a value between 0.5 and 30 °, more preferably between 1 and 20 °, even more preferably between 1 and 10 °, even more preferably between 2 and 9 °, most preferably between 4 and 8 °. In a preferred embodiment the angle alpha has a value of about 1 °, 2 °, 3 °, 4 °, 5 °, 6 °, 7 °, 8 °, 9 °, 10 °, 11 °, 12 °, 13 °, 14 °, 15 °, 16 °, 17 °, 18 °, 19 °, 20 °, 21 °, 22 °, 23 °, 24 °, 25 °, 26 °, 27 °, 28 °, 29 ° or 30 °.

In another embodiment, the absorbent article comprises at least one rear side panel, preferably two rear side panels, of which the upper edge makes a non-zero angle alpha with the transverse axis, whereby the non-zero angle alpha preferably has a value between 0.5 and 30 °, more preferably between 1 and 20 °, even more preferably between 1 and 10 °, even more preferably between 2 and 9 °, most preferably between 4 and 8 °. In a preferred embodiment the angle alpha has a value of about 1 °, 2 °, 3 °, 4 °, 5 °, 6 °, 7 °, 8 °, 9 °, 10 °, 11 °, 12 °, 13 °, 14 °, 15 °, 16 °, 17 °, 18 °, 19 °, 20 °, 21 °, 22 °, 23 °, 24 °, 25 °, 26 °, 27 °, 28 °, 29 ° or 30 °.

In yet another embodiment, the absorbent article comprises at least one front side panel and at least one rear side panel, preferably two front side panels and two rear side panels, of which the upper edge makes a non-zero angle alpha with the transverse axis, whereby the non-zero angle alpha preferably has a value between 0.5 and 30 °, more preferably between 1 and 20 °, even more preferably between 1 and 10 °, even more preferably between 2 and 9 °, most preferably between 4 and 8 °. In a preferred embodiment the angle alpha has a value of about 1 °, 2 °, 3 °, 4 °, 5 °, 6 °, 7 °, 8 °, 9 °, 10 °, 11 °, 12 °, 13 °, 14 °, 15 °, 16 °, 17 °, 18 °, 19 °, 20 °, 21 °, 22 °, 23 °, 24 °, 25 °, 26 °, 27 °, 28 °, 29 ° or 30 °.

As mentioned above, the absorbent article can comprise more than one side panel, such as for example two front side panels, two rear side panels, a front and a rear side panel, two front side panels and a rear side panels, two rear side panels and a front side panel, two front side panels and two rear side panels, etc. If the absorbent article comprises more than one side panel, the angle alpha of the upper edge of one side panel does not necessarily have to correspond with the angle alpha of the upper edge of the other side panel(s), i.e. the angle alpha of one side panel can be larger, smaller or equal in size than the angle alpha of the other side panel(s). Comparing for example the angle alpha of the upper edge between a front side panel and a rear side panel, a front side panel and another front side panel or a rear side panel and another rear side panel, the angle alpha can thus be larger, smaller or equal in size. Though not a preferred embodiment, it is also plausible that the upper edge of a side panel can display an angle alpha which is 0 °, on the condition that at least one of the other side panel(s) has an upper edge that displays a non-zero angle alpha with the transverse axis. Preferably, the angle alpha of the upper edge of two front side panels and/or two rear side panels will be equal in size. In another embodiment, the angle alpha of the upper edge of a front side panel will be larger, smaller or equal in size than the angle alpha of the upper edge of a rear side panel. This will allow to optimize the shape of each separate side panel to the intended use and/or user of the absorbent article, i.e. in view of its circumference, whether the user is a man or a female, the intended use during the day or the night, etc.

An absorbent article according to the current invention having at least one side panel of which the upper edge makes a non-zero angle alpha with the transverse axis allows the side panel to fit better around the waist of a wearer, especially when the user of the absorbent article is an adult, which circumference can take larger proportions than, for example, an infant. When the upper edge makes an angle of 0 ° compared to the transverse axis, as now is used in the prior art, this edge tends to "cut", "wound" and/or at least "irritate" the waist of the wearer, providing him or her with an uncomfortable feeling. By providing the upper edge of a side panel with a non-zero angle alpha, allows to side panels to better follow the waistline of the wearer, thus improving the overall wearer comfort of the user.

The front and/or rear side panel preferably has a proximal length and a distal length. "Proximal length" as used herein, refers to the length of the proximal edge between the upper and the lower edge as measured along the longitudinal axis, whereas the "distal length" as used herein, refers to the length of the distal edge between the upper and the lower edge as measured along the longitudinal axis. The side panel further preferably has a side panel width. The "side panel width" as used herein, refers to the width of the front and/or rear side panel measured along the transverse axis between the proximal and the distal edge. Because the upper edge of the front and/or the rear side panel makes a non-zero angle alpha with the transverse axis, the distal length may vary from the proximal length with an upper distance. The term "upper distance" as used herein, refers to the difference in distance as measured along the longitudinal axis between the point where the upper edge crosses the proximal edge and the point where the upper edge crosses the distal edge.

With the definition that the upper edge makes "a non-zero angle alpha with the transverse axis" it is meant that an imaginative straight line between the point where the upper edge crosses the proximal edge and the point where the upper edge crosses the distal edge, makes a non-zero angle with the transverse axis. This by no means implies that the upper edge is necessarily a straight line, as the upper edge can display any type of regular or irregular shape such as a linear shape, curved shape, undulated shape, stepwise shape, zig saw shape, S-shape, etc. and/or can display a symmetry or can be asymmetrical.

The ratio of the upper distance and the side panel width preferably comprises a value between 0.01 and 0.50, more preferably between 0.03 and 0.40, even more preferably between 0.05 and 0.35, even more preferably between 0.10 and 0.25, even more preferably between 0.12 and 0.23, most preferably between 0.15 and 0.19. In a preferred embodiment, the ratio of the upper distance and the side panel width comprises a value of about 0.10, 0.11, 0.12, 0.13, 0.14, 0.15, 0.16, 0.17, 0.18, 0.19, 0.20, 0.21, 0.22, 0.23, 0.24, 0.25, 0.26, 0.27, 0.28, 0.29, 0.30, 0.31, 0.32, 0.33, 0.34 or 0.35.

If the absorbent article comprises more than one side panel, the ratio of the upper distance and the side panel width of one side panel does not necessarily have to correspond with the ratio of the upper distance and the side panel width of the other side panel(s), i.e. the ratio can be larger, smaller or equal in size than the ratio of the other side panel(s). If, for example, the upper distance and the side panel width of one side panel are represented by C1 and A1 respectively, and the upper distance and side panel width of the other side panel are represented by C2 and A2 respectively, the ratio of the upper distance and the side panel width of the one side panel, i.e. C1/A1, can adopt a value between 0<C1/A1≤0.50, more preferably between 0.03≤C1/A1≤0.40, even more preferably between 0.05≤C1/A1≤0.35, and the ratio of the upper distance and the side panel width of the other side panel, i.e. C2/A2, can adopt a value between 0<C2/A2≤0.50, more preferably between 0.03≤C2/A2≤0.40, even more preferably between 0.05≤C2/A2≤0.35. Comparing the ratio of the upper distance and the side panel width for example between a front side panel and a rear side panel, a front side panel and another front side panel or a rear side panel and another rear side panel, the ratio can thus be larger, smaller or equal in size. This will allow to optimize the shape of each separate side panel to the intended use and/or user of the absorbent article.

Preferably, the upper edge makes a non-zero angle alpha with the transverse axis in such a manner that, when comparing the point where the upper edge crosses the proximal edge and the point where the upper edge crosses the distal edge, the point where the upper edge crosses the distal edge is spaced farther away from the front edge or rear edge for respectively a front side panel and a rear side panel when looking along the longitudinal axis than the point where the upper edge crosses the proximal edge. Studies have shown that this type of angle allows a more optimally improved fit of the side panel around the waist of a wearer of the absorbent article.

An absorbent article with a side panel according to the present invention is also advantageous when producing the side panel for the absorbent article. Side panels are typically made by cutting a web material in the desired shape of the side panel after which the side panel is attached to the absorbent article. However, usually some web material is lost during cutting as some pieces of web material are left over which are too small to make new side panels of, hence the leftover web material becomes waste.

An absorbent article with a side panel according to the present invention, whereby the proximal and the distal edge substantially run parallel to each other along the longitudinal axis, makes it easier to produce the side panels, as they can be made in a simple process using an essentially endless layer of a web material which has two substantially parallel opposed length sides, which correspond with the proximal and the distal edge of the side panels to be made, and simply cutting the layer from one length side to the other, whereby the upper edge of two adjacent side panels can be formed simultaneously. To make the upper edge of a side panel with a non-zero angle alpha, the web layer can simply be cut along a certain angle, whereby adjacent side panels will simultaneously form complementary angles. This makes the production process overall more facile and cost effective as less web material is lost during cutting. In a preferred embodiment, the front and/or the rear side panel are made from the same web material without loss of web material when making the side panels, i.e. for multiple absorbent articles, at least one of the side panels is made from the same web material, without any waste web material remaining after production. In this manner, the machine and process can be made much more economical, i.e. the cutting devices become cheaper, require lower investment costs and lower maintenance when comparing prior art technologies.

An absorbent article with a front and/or rear side panel according to the present invention thus allows an improved fit of the side panels around the waist of a wearer, while still allowing the side panels to be produced in a cost effective, facile manner, thus providing an optimal balance between wearing comfort and production cost.

According to an embodiment of the current invention, the upper edge of the front and/or rear side panel displays a point symmetry with respect to a symmetry point located on the upper edge, centrally between the proximal and the distal edge, i.e. substantially in the middle between the proximal and the distal edge. This means that if the upper edge is rotated about this symmetry point by 180 °, it looks substantially identical to the upper edge before this rotation. This is typically observed for substantially straight (linear) upper edges, but also for other types of configurations, such as curved shapes, for example S-shaped edges, stepwise shapes, or other configurations that display the above described point symmetry. Preferably, the upper edge is straight or substantially straight. The point symmetry allows similar side panels to be fabricated within the same line, as, by applying a similar cutting process as described above, the point symmetry allows adjacent side panels to have a similar complimentary shape.

In a preferred embodiment, the lower edge of the front and/or the rear side panel makes an angle beta with the transverse axis. "Beta" or the symbol "β" are used herein as synonyms. With the definition of the lower edge makes "an angle beta with the transverse axis" it is meant that an imaginative straight line between the point where the lower edge crosses the proximal edge and the point where the lower edge crosses the distal edge, makes an angle with the transverse axis. This by no means implies that the upper edge is necessarily a straight line, as the lower edge can display any type of regular or irregular shape such as a linear shape, curved shape, undulated shape, stepwise shape, zig saw shape, S-shape, etc. and/or can display a symmetry or can be asymmetrical. Nor does it mean that the angle beta cannot be 0 °. However, preferably, the angle beta is not 0 °.

The angle beta preferably has a value between 1 and 50 °, more preferably between 2 and 40 °, even more preferably between 3 and 30 °, even more preferably between 4 and 33 °, even more preferably between 5 and 28 °, most preferably between 6 and 27 °. In a preferred embodiment the angle beta has a value of about 5 °, 6 °, 7 °, 8 °, 9 °, 10 °, 11 °, 12 °, 13 °, 14 °, 15 °, 16 °, 17 °, 18 °, 19 °, 20 °, 21 °, 22 °, 23 °, 24 °, 25 °, 26 °, 27 °, 28 °, 29 °, 30 °, 31 °, 32 °, 33 °, 34 °, 35 °, 36 °, 37 °, 38 °, 39 °, 40 °, 41 °, 42 °, 43 °, 44 ° or 45 °. The angle beta may be smaller or larger than the angle alpha or may have the same value as the angle alpha. In a preferred embodiment, the angle beta is larger than the angle alpha.

By providing the lower edge of the front and/or the rear side panel with an angle beta with the transverse axis allows to tune the leg opening of the absorbent article created by the side panels and allows a better fit of the side panel around the legs of the wearer.

If the absorbent article comprises more than one side panel, the angle beta of the lower edge of one side panel does not necessarily have to correspond with the angle beta of the lower edge of the other side panel(s), i.e. the angle beta of one side panel can be larger, smaller or equal in size than the angle beta of the other side panel(s). Comparing for example the angle beta of the lower edge between a front side panel and a rear side panel, a front side panel and another front side panel or a rear side panel and another rear side panel, the angle beta can thus be larger, smaller or equal in size. Preferably, the angle beta of the lower edge of two front side panels and/or two rear side panels will be equal in size. In a preferred embodiment, the angle beta of the lower edge of a front side panel will be smaller than the angle beta of the lower edge of a rear side panel. This will allow to optimize the shape of each separate side panel to the intended use and/or user of the absorbent article.

Preferably, the lower edge of the front and/or the rear side panel makes an angle beta in such a manner that, when comparing the point where the lower edge crosses the proximal edge and the point where the lower edge crosses the distal edge, the point where the lower edge crosses the distal edge is spaced closer to the front edge or rear edge for respectively a front side panel and a rear side panel when looking along the longitudinal axis than the point where the upper edge crosses the proximal edge. Studies have shown that this type of angle allows a more optimally improved fit of the side panel around the legs of a wearer of the absorbent article.

In one embodiment, the lower edge displays a point symmetry with respect to a symmetry point located on the lower edge, centrally between the proximal and the distal edge. This means that if the lower edge is rotated about this symmetry point by 180 °, it looks substantially identical to the lower edge before this rotation. This is typically observed for substantially straight (linear) lower edges, but also for other types of configurations, such as curved shapes, for example S-shaped edges, stepwise shapes, or other configurations that display the above described point symmetry. Similar as for the upper edge, the point symmetry allows similar side panels to be fabricated within the same line, as, by applying a similar cutting process as described above, the point symmetry allows adjacent side panels to have a similar complimentary shape. In another embodiment, both the upper edge and the lower edge display a point symmetry with respect to a symmetry point located on that upper and lower edge, centrally between the proximal and the distal edge, which allows identically shaped side panels to be fabricated within the same line.

Preferably, the lower edge of the front and/or the rear side panel is straight or substantially straight. More preferably, both the lower edge and the upper edge of the front and/or the rear side panel are straight or substantially straight. In a preferred embodiment, the front and/or the rear side panel have a substantially trapezoidal shape.

When the lower edge of the front and/or the rear side panel makes an angle beta with the transverse axis, the distal length may vary from the proximal length with a lower distance. The term "lower distance" as used herein, refers to the difference in distance as measured along the longitudinal axis between the point where the lower edge crosses the proximal edge and the point where the lower edge crosses the distal edge. The lower distance may be smaller, larger or equal in size than the upper distance and can be adapted depending on whether it concerns a front or a rear side panel. In a preferred embodiment, the lower distance will be larger than or equal to the upper distance, i.e. B≥C, if the upper distance is represented by C and the lower distance is represented by B, which according to studies, provided a more improved fit of the side panel(s) around the waist of the wearer.

The ratio of the lower distance and the side panel width preferably comprises a value between 0 and 1, more preferably between 0 and 0.50, more preferably between 0.10 and 0.40, even more preferably between 0.20 and 0.30, more preferably between 0.21 and 0.29, most preferably between 0.22 and 0.28. In a preferred embodiment, the ratio of the upper distance and the side panel width comprises a value of about 0.20, 0.21, 0.22, 0.23, 0.24, 0.25, 0.26, 0.27, 0.28, 0.29, 0.30, 0.31, 0.32, 0.33, 0.34 or 0.35.

If the absorbent article comprises more than one side panel, the ratio of the lower distance and the side panel width of one side panel does not necessarily have to correspond with the ratio of the lower distance and the side panel width of the other side panel(s), i.e. the ratio can be larger, smaller or equal in size than the ratio of the other side panel(s). If, for example, the lower distance and the side panel width of one side panel are represented by B1 and A1 respectively, and the lower distance and side panel width of the other side panel are represented by B2 and A2 respectively, the ratio of the lower distance and the side panel width of the one side panel, i.e. B1/A1, can adopt a value between 0≤B1/A1≤1, more preferably between 0≤B1/A1≤0.50, even more preferably between 0.1≤B1/A1≤0.4, and the ratio of the lower distance and the side panel width of the other side panel, i.e. B2/A2, can adopt a value between 0≤B2/A2≤1, more preferably between 0≤B2/A2≤0.50, even more preferably between 0.1≤B2/A2≤0.4. Comparing the ratio of the lower distance and the side panel width for example between a front side panel and a rear side panel, a front side panel and another front side panel or a rear side panel and another rear side panel, the ratio can thus be larger, smaller or equal in size. This will allow to optimize the shape of each separate side panel to the intended use and/or user of the absorbent article.

In a preferred embodiment, the side panel width of the front and/or the rear side panel has a value between 25 and 350 mm, more preferably between 50 and 300 mm, even more preferably between 70 and 270 mm, most preferably between 110 and 210 mm.

If the absorbent article comprises more than one side panel, the side panel width of one side panel does not necessarily have to correspond with the side panel width of the other side panel(s), i.e. the side panel width can be larger, smaller or equal in size than the side panel width of the other side panel(s).). If, for example, the side panel width of one side panel is represented by A1, and the side panel width of the other side panel is represented by A2, the ratio A1 and A2 can adopt a value between 0<A1/A2≤1. Comparing the side panel width for example between a front side panel and a rear side panel, a front side panel and another front side panel or a rear side panel and another rear side panel, the side panel width can thus be larger, smaller or equal in size. This will allow to optimize the shape of each separate side panel to the intended use and/or user of the absorbent article.

In a preferred embodiment, the distal length of the front and/or the rear side panel has a value between 50 and 300 mm, more preferably between 100 and 250 mm, even more preferably between 150 and 200 mm, even more preferably between 160 and 190 mm, most preferably between 172 and 187 mm.

If the absorbent article comprises more than one side panel, the distal length of one side panel does not necessarily have to correspond with the distal length of the other side panel(s), i.e. the distal length of one side panel can be larger, smaller or equal in size than the distal length of the other side panel(s). If, for example, the distal length of one side panel is represented by F1, and the distal length of the other side panel is represented by F2, the ratio F1 and F2 can adopt a value between 0<F1/F2≤1. Comparing the distal length for example between a front side panel and a rear side panel, a front side panel and another front side panel or a rear side panel and another rear side panel, the distal length can thus be larger, smaller or equal in size. All this will allow to optimize the shape of each separate side panel to the intended use and/or user of the absorbent article. In a preferred embodiment, if the absorbent article contains both a front side panel and a rear side panel, the distal length of a front side panel will be larger than the distal length of a rear side panel. Seeing the front side panels of the absorbent article are typically positioned first around the waist of a wearer, a larger distal length of the front side panel will facilitate this positioning.

In a preferred embodiment, the proximal length of the front and/or the rear side panel has a value between 50 and 400 mm, more preferably between 100 and 350 mm, even more preferably between 150 and 325 mm, even more preferably between 200 and 300 mm, most preferably between 225 and 295 mm.

If the absorbent article comprises more than one side panel, the proximal length of one side panel does not necessarily have to correspond with the proximal length of the other side panel(s), i.e. the proximal length can be larger, smaller or equal in size than the proximal length of the other side panel(s). If, for example, the proximal length of one side panel is represented by G1, and the proximal length of the other side panel is represented by G2, the ratio G1 and G2 can adopt a value between 0<G1/G2≤1. Comparing the proximal length for example between a front side panel and a rear side panel, a front side panel and another front side panel or a rear side panel and another rear side panel, the proximal length can thus be larger, smaller or equal in size. This will allow to optimize the shape of each separate side panel to the intended use and/or user of the absorbent article.

In a preferred embodiment, the upper distance of the front and/or the rear side panel has a value between 1 mm and 150 mm, more preferably between 1 and 100 mm, even more preferably between 5 and 50 mm, even more preferably between 10 and 30 mm, most preferably between 12 and 27 mm.

If the absorbent article comprises more than one side panel, the upper distance of one side panel does not necessarily have to correspond with the upper distance of the other side panel(s), i.e. the upper distance can be larger, smaller or equal in size than the upper distance of the other side panel(s). If, for example, the upper distance of one side panel is represented by C1, and the upper distance of the other side panel is represented by C2, the ratio C1 and C2 can adopt a value between 0<C1/C2≤1. Comparing the upper distance for example between a front side panel and a rear side panel, a front side panel and another front side panel or a rear side panel and another rear side panel, the upper distance can thus be larger, smaller or equal in size. This will allow to optimize the shape of each separate side panel to the intended use and/or user of the absorbent article. Preferably, the upper distance of a front side panel will be larger than the upper distance of a rear side panel. Studies have shown that a larger upper distance in the front side panel will facilitate the positioning of the absorbent article around the waist of the wearer.

In a preferred embodiment, the lower distance of the front and/or the rear side panel has a value between 1 and 200 mm, more preferably between 5 and 150 mm, even more preferably between 10 and 60 mm, even more preferably between 20 and 40 mm, most preferably between 23 and 37 mm.

If the absorbent article comprises more than one side panel, the lower distance of one side panel does not necessarily have to correspond with the lower distance of the other side panel(s), i.e. the lower distance can be larger, smaller or equal in size than the lower distance of the other side panel(s). If, for example, the lower distance of one side panel is represented by B1, and the lower distance of the other side panel is represented by B2, the ratio B1 and B2 can adopt a value between 0<B1/B2≤1. Comparing the lower distance for example between a front side panel and a rear side panel, a front side panel and another front side panel or a rear side panel and another rear side panel, the lower distance can thus be larger, smaller or equal in size. This will allow to optimize the shape of each separate side panel to the intended use and/or user of the absorbent article. Preferably, the lower distance of a front side panel will be smaller than or equal to the lower distance of a rear side panel. Studies have shown that this configuration will facilitate the positioning of the absorbent article around the legs of the wearer.

In a preferred embodiment, the distal length of a front side panel and/or rear side panel will be larger than or equal to the sum of the upper and the lower distance. This prevents that the side panel becomes too narrow or too broad as it extends from the side edges of the chassis towards the distal edge of the side panel, which would result in an uncomfortable feeling for the user when wearing the absorbent article.

The front and/or side panel can comprise any type of material known in the art. Preferably, the front and/or the rear side panel are formed from or comprise a non-woven material. In case the absorbent article comprises both a front side panel and a rear side panel, the rear side panel can comprise the same or a different type of material than the front side panel. Preferably, the rear side panel, if present, is elasticized or comprises an elasticized part.

In a preferred embodiment, the absorbent article comprises two front side panels, one on each transversely opposed side edge of the front region, and two rear side panels, one on each transversely opposed side edge of the rear region, whereby the rear side panels preferably comprise at least one fastening means attached to them which are able to connect to the front side panels in a refastenable manner, hereby joining together the front region and the rear region, i.e. thus forming two leg openings to accommodate the legs of the wearer and a waist opening to apply to the waist of the wearer. In a preferred embodiment, each rear side panel comprises two fastening means attached to them which are able to connect to the front side panels in a refastenable manner. The fastening means can be provided to the rear side panels in various configurations well known in the art. In a preferred embodiment, the absorbent article is an adult incontinence diaper.

Preferably, when the rear side panel comprises two fastening means, the fastening means lying closest to the upper edge is spaced from that upper edge by a distance D, as measured along the longitudinal axis, whereas the fastening means lying closest to the lower edge is spaced from that lower edge by a distance E, as measured along the longitudinal axis. The distance E can be larger, smaller or equal in size than the distance D. In a preferred embodiment, the distance E is larger than or equal to the distance D, i.e. E≥D, which allows a more improved fit of the absorbent article once positioned around the waist of the wearer.

The distance E can further be larger, smaller or equal in size than the upper and/or lower distance. In a preferred embodiment, the lower distance will be larger than or equal to the distance E, i.e. B≥E, if the lower distance is represented by B. Likewise, the distance D can be larger, smaller or equal in size than the upper and/or lower distance. All this will allow to optimize the fitting of the absorbent article in view of the intended use and/or user of the absorbent article.

Further, one fastening means does not necessarily have to comprise the same dimensions as the other fastening means. If, for example, the fastening means lying closest to the upper edge comprises a height I, defined as the largest dimension of that fastening means as measured along the longitudinal axis, and the fastening means lying closest to the lower edge comprises a height J, defined as the largest dimension of that fastening means as measured along the longitudinal axis, the height I can be larger, smaller or equal in size than the height J. In a preferred embodiment, however, the height J is equal in size to the height I, i.e. J=I. The height of the fastening means, i.e. height I and/or J can be larger, smaller or equal in size than the distance E and/or the distance D. This will allow to optimize the fitting of the absorbent article in view of the intended use and/or user of the absorbent article. In a preferred embodiment, the height of the fastening means, i.e. height I and/or J will be larger than the distance D and/or E.

The two fastening means are preferably spaced apart from each other by a distance H, as measured along the longitudinal axis. The distance H can be larger, smaller or equal in size than the sum of distance E and D. In a preferred embodiment, the distance H will be larger or equal to the sum of distance D and E, i.e. H≥(E+D). This configuration, whereby the two fastening means are positioned close to the upper and lower edge of the rear side panel, allows a more optimized connection of the rear side panel to the front region of the absorbent article, and allows it to be contained better around the waist of the wearer. The distal length of a side panel is preferably the sum of the distance D and E, the heights I and J and the distance H, or F=D+E+I+J+H, with F representing the distal length, i.e. preferably, the fastening means do not extend beyond the upper and/or lower edge of the side panel.

In a preferred embodiment, the distance D of the rear side panel has a value between 1 mm and 150 mm, more preferably between 1 and 100 mm, even more preferably between 2 and 50 mm, even more preferably between 3 and 30 mm, most preferably between 5 and 20 mm.

If the absorbent article comprises more than one side panel, the distance D of one side panel does not necessarily have to correspond with the distance D of the other side panel(s), i.e. the distance D can be larger, smaller or equal in size than the distance D of the other side panel(s). If, for example, the distance D of one side panel is represented by D1, and the distance D of the other side panel is represented by D2, the ratio D1 and D2 can adopt a value between 0<D1/D2≤1. Comparing the distance D for example between a rear side panel and another rear side panel, the distance D can thus be larger, smaller or equal in size. This will allow to optimize the fitting of the absorbent article in view of the intended use and/or user of the absorbent article.

In a preferred embodiment, the distance E of the rear side panel has a value between 1 and 200 mm, more preferably between 5 and 150 mm, even more preferably between 10 and 80 mm, even more preferably between 20 and 60 mm, most preferably between 25 and 50 mm.

If the absorbent article comprises more than one side panel, the distance E of one side panel does not necessarily have to correspond with the distance E of the other side panel(s), i.e. the distance E can be larger, smaller or equal in size than the distance E of the other side panel(s). If, for example, the distance E of one side panel is represented by E1, and the distance E of the other side panel is represented by E2, the ratio E1 and E2 can adopt a value between 0<E1/E2≤1. Comparing the distance E for example between a rear side panel and another rear side panel, the distance E can thus be larger, smaller or equal in size. This will allow to optimize the fitting of the absorbent article in view of the intended use and/or the user of the absorbent article.

In a preferred embodiment, the distance H of the rear side panel has a value between 1 mm and 400 mm, more preferably between 1 and 300 mm, even more preferably between 2 and 200 mm, even more preferably between 3 and 150 mm, most preferably between 50 and 120 mm.

If the absorbent article comprises more than one side panel, the distance H of one side panel does not necessarily have to correspond with the distance H of the other side panel(s), i.e. the distance H can be larger, smaller or equal in size than the distance H of the other side panel(s). If, for example, the distance H of one side panel is represented by H1, and the distance H of the other side panel is represented by H2, the ratio H1 and H2 can adopt a value between 0<H1/H2≤1. Comparing the distance H for example between a rear side panel and another rear side panel, the distance H can thus be larger, smaller or equal in size. This will allow to optimize the fitting of the absorbent article in view of the intended use and/or user of the absorbent article.

In a preferred embodiment, the height I and/or the height J of the rear side panel has a value between 1 and 200 mm, more preferably between 5 and 150 mm, even more preferably between 10 and 80 mm, even more preferably between 15 and 60 mm, most preferably between 20 and 50 mm.

If the absorbent article comprises more than one side panel, the height I and/or the height J of one side panel does not necessarily have to correspond with the height I and/or the height J of the other side panel(s), i.e. the height I and/or the height J can be larger, smaller or equal in size than the height I and/or the height J of the other side panel(s). If, for example, the height I and/or the height J of one side panel is represented by I1 and/or J1, and the height I and/or the height J of the other side panel is represented by I2 and/or J2, the ratio I1 and I2 and/or the ratio J1 and J2 can adopt a value between 0<I1/I2≤1 and/or 0<J1/J2≤1. Comparing the height I and/or the height J for example between a rear side panel and another rear side panel, the height I and/or the height J can thus be larger, smaller or equal in size. This will allow to optimize the fitting of the absorbent article in view of the intended use and/or user of the absorbent article.

According to another preferred embodiment, the absorbent article comprises two front side panels, one on each transversely opposed side edge of the front region, and two rear side panels, one on each transversely opposed side edge of the rear region, whereby preferably each front side panel and rear side panel, attached to the same side edge of the chassis, are attached to each other so as to form a pant-like absorbent article, i.e. thus forming two leg openings to accommodate the legs of the wearer and a waist opening to apply to the waist of the wearer. The attachment of the front side panel and the rear side panel to each other can be performed in a wide range of configurations well known in the art. In a preferred embodiment, the absorbent article is an adult incontinence pants.

In a second aspect, the current invention provides a side panel for use as a front and/or a rear side panel in an absorbent article as described above, whereby the side panel comprises two substantially parallel opposed length sides and two width sides extending between the two length sides, whereby at least one of the two width sides makes a non-zero angle alpha with an axis perpendicular to the two length sides. The angle alpha preferably has a value between 0.5 and 30 °, more preferably between 1 and 20 °, even more preferably between 1 and 10 °, even more preferably between 2 and 9 °, most preferably between 4 and 8 °. In a preferred embodiment the angle alpha has a value of about 1 °, 2 °, 3 °, 4 °, 5 °, 6 °, 7 °, 8 °, 9 °, 10 °, 11 °, 12 °, 13 °, 14 °, 15 °, 16 °, 17 °, 18 °, 19 °, 20 °, 21 °, 22 °, 23 °, 24 °, 25 °, 26 °, 27 °, 28 °, 29 ° or 30 °.

In one embodiment, the side panel is used as at least one front side panel in an absorbent article, whereby at least one of the two width sides of the front side panel makes a non-zero angle alpha with an axis perpendicular to the two length sides. The non-zero angle alpha hereby preferably has a value between 0.5 and 30 °, more preferably between 1 and 20 °, even more preferably between 1 and 10 °, even more preferably between 2 and 9 °, most preferably between 4 and 8 °. In a preferred embodiment the angle alpha has a value of about 1 °, 2 °, 3 °, 4 °, 5 °, 6 °, 7 °, 8 °, 9 °, 10 °, 11 °, 12 °, 13 °, 14 °, 15 °, 16 °, 17 °, 18 °, 19 °, 20 °, 21 °, 22 °, 23 °, 24 °, 25 °, 26 °, 27 °, 28 °, 29 ° or 30 °.

In another embodiment, the side panel is used as at least one rear side panel in an absorbent article, whereby at least one of the two width sides of the rear side panel makes a non-zero angle alpha with an axis perpendicular to the two length sides. The non-zero angle alpha hereby preferably has a value between 0.5 and 30 °, more preferably between 1 and 20 °, even more preferably between 1 and 10 °, even more preferably between 2 and 9 °, most preferably between 4 and 8 °. In a preferred embodiment the angle alpha has a value of about 1 °, 2 °, 3 °, 4 °, 5 °, 6 °, 7 °, 8 °, 9 °, 10 °, 11 °, 12 °, 13 °, 14 °, 15 °, 16 °, 17 °, 18 °, 19 °, 20 °, 21 °, 22 °, 23 °, 24 °, 25 °, 26 °, 27 °, 28 °, 29 ° or 30 °.

In yet another embodiment, the side panel is used as at least one front and/or rear side panel in an absorbent article, whereby at least one of the two width sides of the front and/or rear side panel makes a non-zero angle alpha with an axis perpendicular to the two length sides. The non-zero angle alpha hereby preferably has a value between 0.5 and 30 °, more preferably between 1 and 20 °, even more preferably between 1 and 10 °, even more preferably between 2 and 9 °, most preferably between 4 and 8 °. In a preferred embodiment the angle alpha has a value of about 1 °, 2 °, 3 °, 4 °, 5 °, 6 °, 7 °, 8 °, 9 °, 10 °, 11 °, 12 °, 13 °, 14 °, 15 °, 16 °, 17 °, 18 °, 19 °, 20 °, 21 °, 22 °, 23 °, 24 °, 25 °, 26 °, 27 °, 28 °, 29 ° or 30 °.

In one embodiment, at least one of the two width sides further displays a point symmetry with respect to a symmetry point located on that width side, centrally between the two length sides.

In a final aspect, the current invention provides a method to produce a side panel as described above, comprising the steps of:
a. providing a layer of a web material which is essentially endless along an x-axis and has two substantially parallel opposed length sides;
b. providing cutting means; and
c. cutting said layer with said cutting means at two distinct places along the x-axis, hereby forming a side panel with two width sides extending between the two length sides.

In particular, at least one of the two width sides hereby makes a non-zero angle alpha with a y-axis, which y-axis is substantially perpendicular to said x-axis. Preferably, the angle alpha has a value between 0.5 and 30 °, more preferably between 1 and 20 °, even more preferably between 1 and 10 °, even more preferably between 2 and 9 °, most preferably between 4 and 8 °. In a preferred embodiment the angle alpha has a value of about 1 °, 2 °, 3 °, 4 °, 5 °, 6 °, 7 °, 8 °, 9 °, 10 °, 11 °, 12 °, 13 °, 14 °, 15 °, 16 °, 17 °, 18 °, 19 °, 20 °, 21 °, 22 °, 23 °, 24 °, 25 °, 26 °, 27 °, 28 °, 29 ° or 30 °.

The side panel made by a method according to the current invention can be used as a front side panel, a rear side panel or a front and rear side panel within the same or different absorbent articles.

In one embodiment, the side panel made by a method according to the current invention can be used as a front side panel, whereby at least one of the two width sides of the front side panel makes a non-zero angle alpha with an axis perpendicular to the two length sides. The non-zero angle alpha hereby preferably has a value between 0.5 and 30 °, more preferably between 1 and 20 °, even more preferably between 1 and 10 °, even more preferably between 2 and 9 °, most preferably between 4 and 8 °. In a preferred embodiment the angle alpha has a value of about 1 °, 2 °, 3 °, 4 °, 5 °, 6 °, 7 °, 8 °, 9 °, 10 °, 11 °, 12 °, 13 °, 14 °, 15 °, 16 °, 17 °, 18 °, 19 °, 20 °, 21 °, 22 °, 23 °, 24 °, 25 °, 26 °, 27 °, 28 °, 29 ° or 30 °.

In another embodiment, the side panel made by a method according to the current invention can be used as a rear side panel, whereby at least one of the two width sides of the rear side panel makes a non-zero angle alpha with an axis perpendicular to the two length sides. The non-zero angle alpha hereby preferably has a value between 0.5 and 30 °, more preferably between 1 and 20 °, even more preferably between 1 and 10 °, even more preferably between 2 and 9 °, most preferably between 4 and 8 °. In a preferred embodiment the angle alpha has a value of about 1 °, 2 °, 3 °, 4 °, 5 °, 6 °, 7 °, 8 °, 9 °, 10 °, 11 °, 12 °, 13 °, 14 °, 15 °, 16 °, 17 °, 18 °, 19 °, 20 °, 21 °, 22 °, 23 °, 24 °, 25 °, 26 °, 27 °, 28 °, 29 ° or 30 °.

In yet another embodiment, the side panel made by a method according to the current invention can be used as front and/or rear side panel, whereby at least one of the two width sides of the front and/or rear side panel makes a non-zero angle alpha with an axis perpendicular to the two length sides. The non-zero angle alpha hereby preferably has a value between 0.5 and 30 °, more preferably between 1 and 20 °, even more preferably between 1 and 10 °, even more preferably between 2 and 9 °, most preferably between 4 and 8 °. In a preferred embodiment the angle alpha has a value of about 1 °, 2 °, 3 °, 4 °, 5 °, 6 °, 7 °, 8 °, 9 °, 10 °, 11 °, 12 °, 13 °, 14 °, 15 °, 16 °, 17 °, 18 °, 19 °, 20 °, 21 °, 22 °, 23 °, 24 °, 25 °, 26 °, 27 °, 28 °, 29 ° or 30 °.

Preferably, cutting step c) is repeated to produce an essentially endless line of side panels adjacent to each other. After the side panels have been cut, each every other side panel in said line is preferably translated along the y-axis, whereas the remaining side panels are preferably rotated over 180 ° along a z-axis, which z-axis is perpendicular to the x-axis and y-axis, and subsequently translated along the y-axis. The thus produced side panels can be used as a front side panel, a rear side panel or a front and rear side panel within the same or different absorbent articles according to the current invention.

The method according to the present invention, allows that, by performing a single cut in the web material, the width sides of two adjacent side panels can be formed simultaneously, thus making the production process overall more facile and cost effective as less web material is lost during cutting. In a preferred embodiment, no web material is lost during cutting, i.e. no waste web material remains after cutting.

In one embodiment, at least one of the two width sides displays a point symmetry with respect to a symmetry point located on that width side, centrally between the two length sides. In a preferred embodiment, both width sides display a point symmetry with respect to a symmetry point located on each width side, centrally between the two length sides. This allows identical side panels to be fabricated within the same line, as the point symmetry allows adjacent side panels to have the same complimentary shape.

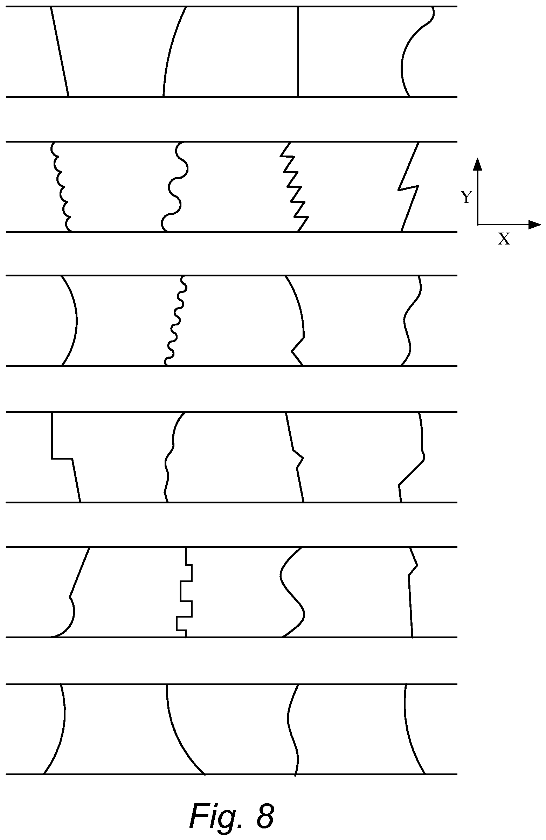

The width sides of the side panels created by the above described method can display any type of regular or irregular shape such as a linear shape, curved shape, undulated shape, stepwise shape, zig saw shape, S-shape, etc. and/or can display a symmetry or can be asymmetrical. Figure 8 provides a few non-exhausting examples of the various shapes the side panel can adapt.

Preferably, the shape of the edges is straight or substantially straight. Preferably, the side panels formed using the method according to the current invention have a substantially trapezoidal shape.

After production of the side panels, the side panels may be attached or joined to the chassis of an absorbent article by any means know in the art.

The present invention will be now described in more details, referring to examples that are not intended to, nor should they be interpreted to, limit the scope of the invention.

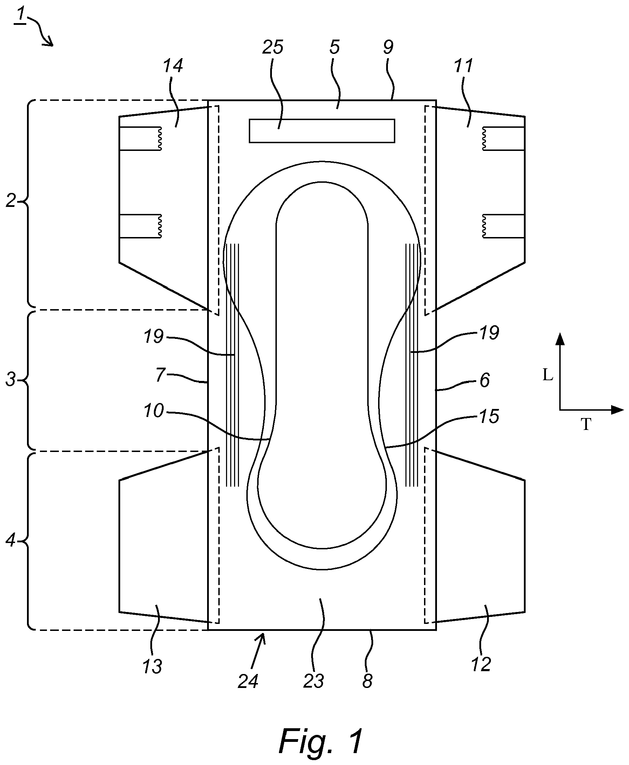

Figure 1 displays an absorbent article according to a preferred embodiment of the current invention, here an adult incontinence diaper (1), displaying a garment-facing side, i.e. the side of the diaper in contact with the undergarment of the wearer during use.

The diaper (1) is shown in its flat out, un-contracted state. The chassis (5) of the diaper (1) in Figure 1 comprises the main body of the diaper (1). The chassis (5) comprises an outer covering including a liquid pervious topsheet (24) and a liquid impervious backsheet (23). The chassis (5) includes an absorbent structure (15) encased between the topsheet (24) and the backsheet (23).

One end portion of the diaper (1) is configured as a front region (4) of the diaper (1). The opposite end portion is configured as a rear region (2) of the diaper (1). An intermediate portion of the diaper (1) is configured as a crotch region (3), which extends longitudinally between the front and rear regions (2) and (4). The regions (2) and (4) may include elastic elements such that they gather about the waist of the wearer to provide improved fit and containment (e.g. elastic waist feature (25)). The crotch region (3) is that portion of the diaper (1) which, when the diaper (1) is worn, is generally positioned between the wearer's legs. The diaper (1) is depicted with its longitudinal axis L and its transverse axis T. The periphery of the diaper (1) is defined by the outer edges of the diaper (1) in which the longitudinal side edges (6) and (7) run generally parallel to the longitudinal axis L of the diaper (1) and the front edge (8) and rear edge (9) run between the longitudinal side edges (6) and (7) generally parallel to the transverse axis T of the diaper. The various components within the diaper (1) may be bound, joined or secured by any method known in the art, for example by adhesives in uniform continuous layers, patterned layers or arrays of separate lines, spirals or spots. The topsheet (24), the backsheet (23), the absorbent structure (15) and other components may be assembled in a variety of well-known configurations and are well known in the art. The chassis (5) can further include other elements well known in the art, such as elasticized leg cuffs (19), containment cuffs (not shown) or elastic waistbands (25).

The backsheet (23) covers the absorbent structure (15) and preferably extends beyond the absorbent structure (15) toward the longitudinal side edges (6) and (7) and the front edge (8) and rear edge (9) of the diaper (1) and may be joined with the topsheet (24). The backsheet (23) prevents the bodily exudates absorbed by the absorbent structure (15) and contained within the diaper (1) from soiling other external articles that may contact the wearer, such as bed sheets and undergarments. In preferred embodiments, the backsheet (23) is substantially impervious to bodily exudates and comprises a laminate of a nonwoven and a thin plastic film such as a thermoplastic film. The backsheet (23) may comprise breathable materials that permit vapor to escape from the diaper (1) while still preventing bodily exudates from passing through the backsheet (23). It may be semi-rigid, non-elastic and can be made fully or partially elasticized and include backing. The backsheet (23) may be assembled in a variety of well-known configurations and are well known in the art.

The diaper (1) comprises a topsheet (24) that is preferably soft, compliant, exhibits good strikethroughs and has a reduced tendency to rewet from the liquid absorbent material. The topsheet (24) is placed in close proximity to the skin of the wearer when the diaper (1) is worn. In this way, such topsheet (24) permits bodily exudates to rapidly penetrate it so as to flow toward the absorbent structure (15) more quickly, but preferably not allowing such bodily exudates to flow back through the topsheet (24). The topsheet (24) may be constructed from any one of a wide range of liquid and vapor permeable, preferably hydrophilic, materials. The upper and lower surface of the topsheet (24) may be treated differently and may for instance include a surfactant on the upper surface so as to facilitate liquid transfer there through, especially at a central zone or area of the topsheet (24) located over the absorbent structure (15), and for instance include a hydrophobic agent on the lower surface to minimize the liquid contained within the absorbent core from contact wetting the topsheet (24) thereby reducing rewet values. The topsheet (24) may also be coated with a substance having rash preventing or rash reducing properties (e.g. aloe vera). The topsheet (24) covers substantially the entire wearer facing area of the diaper (1), including substantially all of the front region (4), rear region (2), and crotch region (3). The topsheet (24) may be formed from a single or multiple different materials which vary across the width of the topsheet (24). Such a multiple piece design allows for creation of preferred properties and different zones of the topsheet (24). The topsheet (24) can be semi-rigid, non-elastic and can be made fully or partially elasticized. The topsheet (24) may be assembled in a variety of well-known configurations and are well known in the art.

The absorbent structure (15) in Figure 1 generally is disposed between the topsheet (24) and the backsheet (23). The absorbent structure (15) may comprise any absorbent material that is generally compressible, conformable, non-irritating to the wearer's skin, and capable of absorbing and retaining bodily exudates. The absorbent structure (15) may comprise a wide variety of liquid absorbent materials commonly used in absorbent articles such as fluff pulp, which is generally referred to as airlaid. Examples of other suitable absorbent materials include creped cellulose wadding; melt blown polymers; chemically stiffened, modified or cross-linked cellulosic fibres; tissue, including tissue wraps and tissue laminates; absorbent foams; absorbent sponges; absorbent polymer materials; absorbent gelling materials; or any other known absorbent materials or combinations of materials. The absorbent structure (15) may further comprise non-liquid absorbent materials, such as adhesives, binders, plastics, waxes, oils and the like. The absorbent structure (15) may be configured to extend substantially the full length and/or width of the diaper (1). However, alternatively the absorbent structure (15) may be not coextensive with the entire diaper (1) and is limited to certain regions of the diaper (1) such as for instance the crotch region (3). The absorbent structure (15) can extend to the edges of the diaper (1) and the absorbent material can be concentrated in the crotch region (3) or another target zone of the diaper (1). Particles used in the absorbent structure (15) can be a combination of absorbent material, preferably comprising absorbent polymer material, and skin care particles such as ion exchange resins, deodorant, anti-microbial agents, binder particles, or other beneficial particles. The diaper (1) may also employ additional layers known in the art including an acquisition layer or surge layer, preferably situated between the topsheet and the absorbent core and highloft and/or coverstock layers. This serves to slow down the flow so that the liquid has adequate time to be absorbed by the absorbent core. The absorbent structure (15) can comprise one or more absorbent cores. The diaper (1) in Figure 1 for example comprises an extra absorbent core (10).

The diaper (1) may also utilize a pair of containment walls or cuffs (not shown). Such cuff is typically a longitudinally extending wall structure preferably positioned on each side of the absorbent structure (15) and spaced laterally from the longitudinal axis L. The longitudinal ends of the cuffs may be attached or joined, for example, to the topsheet (24) in the front and rear regions (4) and (2). Preferably, the ends of the cuffs are tacked down inwardly and attached, for example, by adhesive or sonic bonding to the lower structure. Such a construction effectively biases the cuffs inwardly and is generally considered to cause the cuffs to exhibit improved leakage prevention properties. Preferably, the cuffs are equipped with elastic members (not shown), which extend along a substantial length of the cuffs. In a common application, the elastic members are placed within the cuffs, preferably at the top of the cuff while in a stretched condition and then glued or sonic bonded to the cuff at least at their ends. When released or otherwise allowed relaxing, the elastic members retract inwardly. When the diaper (1) is worn, the elastic members function to contract the cuffs about the buttocks and the thighs of the wearer in a manner, which forms a seals between the diaper (1), the buttocks and the thighs. The cuffs may be assembled in a variety of well-known configurations and are well known in the art. Furthermore, waistbands employing elastic members can be positioned along the transverse portion of the diaper (1), either in the front region (not shown), rear region (25) or both, so that when worn, the waistband is positioned along the waist of the wearer. Generally, the waistband preferably creates a seal against the waist so that bodily exudates do not leak from the regions between the elastic waistband and the waist of the wearer. Although the bodily exudates are primarily absorbed by the absorbent materials within the diaper (1), the seal is important considering the assault of liquid by the wearer may overwhelm the absorption rate capacity of the absorbent structure (15). Hence, the waistband contain the liquid while it is being absorbed, they are well known in the art.

The absorbent article such as a diaper (1) may also include such other features, components and elements as are known in the art including waist cap features, elastics and the like to provide better fit, containment and aesthetic characteristics. These features may be assembled in a variety of well-known configurations and are well known in the art.

According to the embodiment of Figure 1, the front and rear regions (2) and (4) each comprise a pair of front side panels (12) (13) and a pair of rear side panels (11) (14) extending beyond the side edges (6) and (7) of the chassis (5) in a transverse direction. These side panels (11) (12) (13) (14) may be attached or joined to the chassis (5) by any means know in the art. In a preferred embodiment of the present invention, the side panels (11) (14) positioned in the rear region (2) are flexible, extensible and/or elastic in at least the lateral direction (i.e., elasticized side panels), in another embodiment the side panels (11) (12) (13) (14) are non-elastic, semi-rigid, rigid and/or stiff.

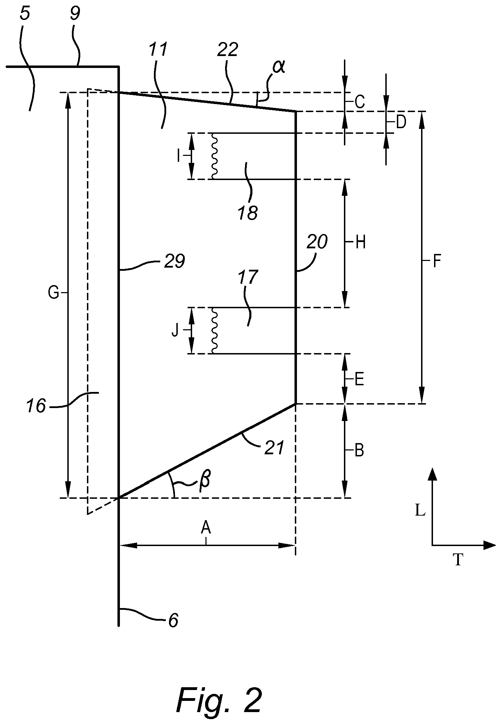

Figure 2 displays a more detailed view of one of the two rear side panels of Figure 1. The rear side panel (11) has a proximal edge (29) and a distal edge (20) which are transversely opposed and run substantially parallel to each other along the longitudinal axis L. The proximal edge (29) according to this embodiment is the edge closest to the side edge (6) of the chassis (5) from which the rear side panel (11) extends in transverse direction and the distal edge (20) is spaced farthest away from said side edge (6). The rear side panel (11) further comprises an upper edge (22) and a lower edge (21), the upper edge (22) being located closer to the rear edge (9) of the chassis (5) than the lower edge (21). The rear side panel (11) is attached to the chassis (5) in an attachment zone (16). The upper edge (22) of the rear side panel (11) makes a non-zero angle alpha (α) with the transverse axis T. According to this embodiment, the lower edge (21) of the rear side panel (11) also makes a non-zero angle beta (β) with the transverse axis T, which angle beta is larger than the angle alpha.

The upper (22) and the lower edge (21) according to this embodiment are substantially straight, thus resulting in a side panel (11) with a substantially trapezoidal shape. The side panel has a proximal length (G), being the length of the proximal edge (29) between the upper (22) and the lower edge (21) as measured along the longitudinal axis L and a distal length (F), being the length of the distal edge (20) between the upper (22) and the lower edge (21) as measured along the longitudinal axis L, as well as a side panel width (A), being the width of the side panel (11) measured along the transverse axis T between the proximal (29) and the distal edge (20). Because of the angle alpha (α) and beta (β), the distal length (F) varies from the proximal length (G) with an upper distance (C), being the difference in distance as measured along the longitudinal axis between the point where the upper edge (22) crosses the proximal edge (29) and the point where the upper edge (22) crosses the distal edge (20); and a lower distance (B), being the difference in distance as measured along the longitudinal axis between the point where the lower edge (21) crosses the proximal edge (29) and the point where the lower edge (21) crosses the distal edge (20).

The ratio of the upper distance (C) and the side panel width (A) according to this embodiment comprises a value between 0.17 and 0.18. The ratio of the lower distance (B) and the side panel width (A) according to this embodiment comprises a value between 0.24 and 0.25.

Referring to Figure 1 and 2, the rear side panels (11) (14) according to this embodiment comprise two fastening means (17) and (18) in order to keep the diaper (1) in place about the wearer, whereby at least a portion of the rear region (2) is attached by fastening means (17) and (18) to at least a portion of the front region (4), preferably to form leg openings and an absorbent article waist. The fastening means (18) lying closest to the upper edge (22) is spaced from that upper edge by a distance (D), whereas the fastening means (17) lying closest to the lower edge (21) is spaced from that lower edge by a distance (E). In this embodiment, the distance (E) is larger than the distance (D). The fastening means lying closest to the upper edge (18) further comprises a height (I), defined as the largest dimension of that fastening means as measured along the longitudinal axis, and the fastening means lying closest to the lower edge (17) comprises a height (J), defined as the largest dimension of that fastening means as measured along the longitudinal axis. In this embodiment, the height (J) is equal in size to the height (I). The two fastening means (17) and (18) are spaced apart from each other by a distance (H), as measured along the longitudinal axis. In this embodiment, the distance (H) is larger than the sum of distance (D) and (E). Finally, the distal length (F) of the side panel (11) according to this embodiment is the sum of the distance (D) and (E), the heights (I) and (J) and the distance (H).

The two fastening means (17) and (18) may comprise the same or a different type of material. The fastening means (17) and (18) may be adhesive, mechanical fasteners, hook and loop features, conceivable strings and/or combinations thereof, i.e., anything that will secure one end of the diaper (1) to the longitudinally opposite end of the diaper (1). The fastening means (17) and (18) may also be co-adhesive such that they adhere to each other but not other materials. Fastening means (17) and (18) and any component thereof may include any material suitable for such a use, including but not limited to plastics, films, foams, non-woven webs, woven webs, paper, laminates, fibre reinforced plastics and the like, or combinations thereof. It may be preferable that the materials making up the fastening means (17) and (18) are flexible, extensible and/or elastic, allowing them to better conform to the shape and movements of the body and thus, reduces the likelihood that the fastening system will irritate or injure the wearer's skin. Preferably, the diaper (1) is affixed to the wearer by tape fasteners (17) and (18) which are permanently affixed to the rear side panels (11) (14). Tape fasteners are contacted with the transversely opposite rear side panels (11) (14), attached and extending from the chassis (5), where they remain affixed due to the binding compound applied to the fasteners. Alternatively, the absorbent article may be pants and the like. In this configuration, the absorbent article may or may not have tape fasteners. Specific disposability tapes may however also be provided on such absorbent articles. All fastening means may be assembled in a variety of well-known configurations and are well known in the art.

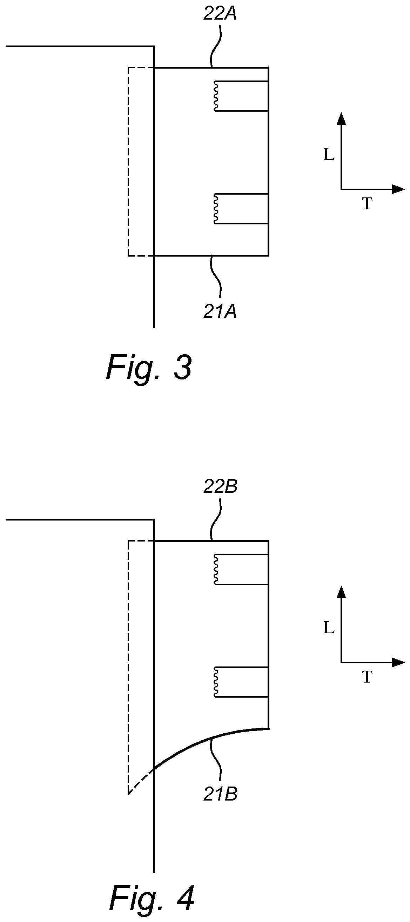

Figure 3 and 4 display schematic views of a rear side panel according to the prior art, with an upper edge (22A/22B) which makes an angle of 0 ° with the transverse axis T. The lower edge can have different configurations, such as straight (21A) or curved (21B). Using a configuration whereby the upper edge makes a non-zero angle (α) with the transverse axis, such as for example depicted in Figure 2, allows a much better fit of the side panels around the waist of a wearer than in the case of using prior art side panels, for example depicted in Figure 3 and 4, as studies have shown that the side panels allow to better follow the waistline of the wearer, especially when that wearer is an adult with a larger circumference, thus improving the overall wearer comfort of the user.

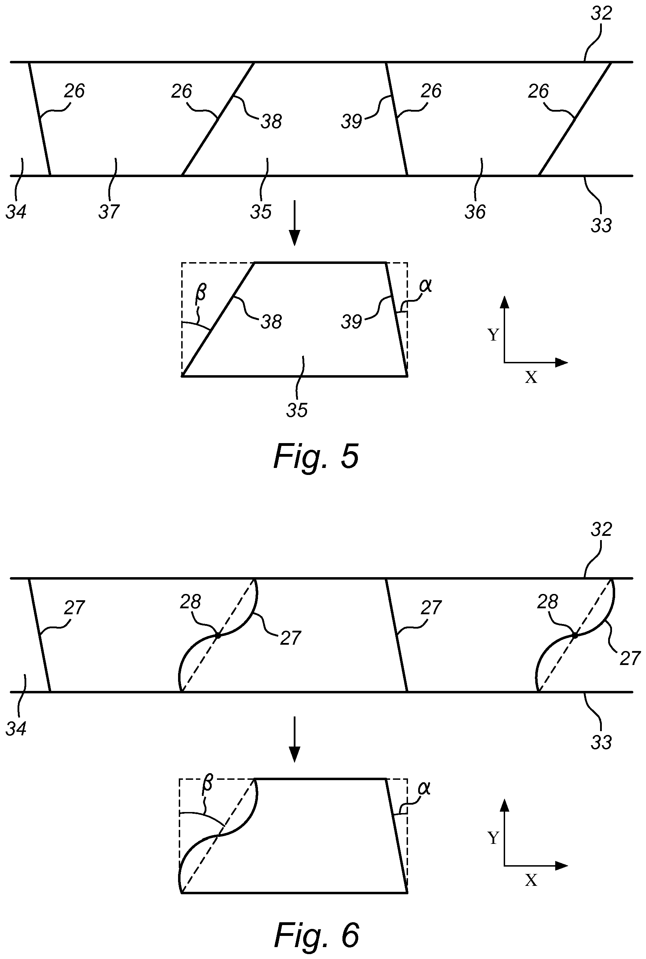

Figure 5 and 6 display schematic representations of methods to make side panels according to two different embodiments of the current invention.

Figure 5 and 6 both display a layer of a web material (34) which is essentially endless along an x-axis and has two substantially parallel opposed length sides (32) and (33). These two substantially parallel opposed length sides will form the proximal and the distal edge of the side panels which will be produced during the method. Referring to Figure 5, to produce one side panel (35), the layer is cut at two distinct places along the x-axis as indicated by the cutting lines (26), which will form the width sides (38) and (39) of a side panel (35). These width sides will form the upper edge and lower edge of the side panels. The width sides (38) and (39) make a non-zero angle alpha (α), hereby forming the upper edge, and an angle beta (β), hereby forming the lower edge, with a y-axis, which y-axis is substantially perpendicular to the x-axis.

The advantage of the current method is that, when making two cutting lines (26) in the web material (34) to make one side panel (35), the width sides (38) and (39) of side panel (35) also form the width sides of two other side panels (36) and (37), which are simultaneously formed during cutting and, in this embodiment, display the same angles alpha (α) and beta (β) as for side panel (35). This way, using one layer of a web material (34), several side panels can be produced with little to no loss of web material.

The cutting lines of Figure 5 are depicted as straight lines, however, the cutting lines (27) can display any type of regular or irregular shape such as a linear shape, curved shape, undulated shape, stepwise shape, zig saw shape, S-shape, etc. and/or can display a symmetry or can be asymmetrical. Figure 8 provides a few non-exhausting examples of the various shapes the width sides of the side panel can adapt.

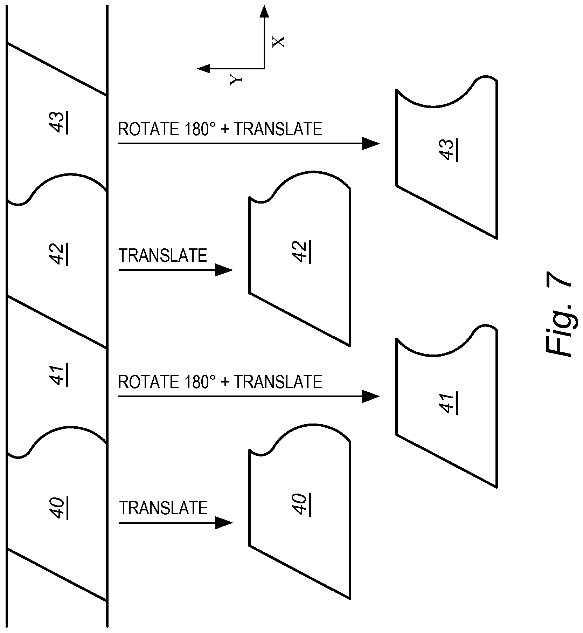

In the embodiment of Figure 6, for example, one of the width sides is curved (S-shaped). The curved line hereby displays point symmetry in a symmetry point (28) located on the width side created by the cutting line (27), centrally between the two length sides (32) and (33). This causes the side panels, which are produced during this cutting process, to adapt identical shapes, as the point symmetry allows adjacent side panels to have the same complimentary shape. However, the width sides formed during the cutting process do not necessarily have to display point symmetry with respect to a symmetry point located on the width side, centrally between the two length sides. As depicted in Figure 7, one of the cutting lines is straight, whereas the other has a non-symmetrical curved shape. This results in the formation of two different types of side panels, whereby, as depicted in Figure 7, side panel 40 and 42 have the same shape, which is different from the shape of side panels 41 and 43. After the side panels are cut into an essentially endless line of side panels adjacent to each other, each every other side panel in the line, in this case for example side panel 40 and 42, is translated along the y-axis and the remaining side panels, in this case for example side panel 41 and 43, are rotated over 180 ° along a z-axis, which z-axis is perpendicular to the x-axis and y-axis, and subsequently translated along the y-axis, which allow to produce separate side panels, i.e. 40, 41, 42 and 43, which can be used in an absorbent article. Each of the thus produced side panels can, for example, be used as a front side panel, a rear side panel or a front and rear side panel within the same or different absorbent articles. Side panels 40 and 42, for example, can be used as two front side panels, whereas side panels 41 and 43, for example, can be used as rear side panels, or vice versa.

As depicted in Figure 8, the cutting lines made in the web material are not restricted two only two different types of shapes. By applying various different cutting lines, numerous amounts of different side panels can be produced using a single essentially endless web layer, without loss of web material during the cutting process.

It is supposed that the present invention is not restricted to any form of realization described previously and that some modifications can be added to the presented example of fabrication without reappraisal of the appended claims.

## Claims

1. A disposable absorbent incontinence article (1) comprising: a liquid permeable topsheet (24), a liquid impermeable backsheet (23) and an absorbent core (15) positioned in between the topsheet (24) and the backsheet (23), whereby the topsheet (24), backsheet (23) and absorbent core (15) form a chassis (5) of the absorbent article (1); the absorbent article (1) further comprising a front region (4), a rear region (2) and a crotch region (3), said crotch region (3) interconnecting the front (4) and the rear (2) region; a longitudinal axis (L) which extends from the front region (4) over the crotch (3) region to the rear (2) region, and a transverse axis (T) which is generally perpendicular to the longitudinal axis (L); whereby the chassis (5) comprises two transversely opposed side edges (6, 7) substantially extending along the longitudinal axis (L), and a front edge (8) and a rear edge (9) at respectively the front region (4) and rear region (2), which front edge (8) and rear edge (9) substantially extend along the transverse axis (T); the absorbent article (1) further comprising at least one front side panel (12, 13) attached to the front region (4) and/or at least one rear side panel (11, 14) attached to the rear region (2), whereby said front (12, 13) and/or rear (11, 14) side panel comprises a proximal edge (29) and a distal edge (20) which are transversely opposed, and an upper edge (22) and a lower edge (21), each extending between the proximal (29) to the distal (20) edge, the upper edge (22) being located closer to the front edge (8) or rear edge (9) of the chassis (5) than the lower edge (21) for respectively a front side panel (12, 13) and a rear side panel (11, 14); wherein the proximal (29) and the distal (20) edge substantially run parallel to each other along the longitudinal axis (L) and whereby the upper edge (22) of the front (12, 13) and/or the rear (11, 14) side panel makes a non-zero angle alpha with the transverse axis (T), whereby the lower edge (21) of the front (12, 13) and/or the rear (11, 14) side panel makes an angle beta with the transverse axis (T), **characterized in that** the angle beta is larger than the angle alpha.

2. Absorbent article (1) according to claim 1, whereby the angle alpha has a value between 0.5 and 30 °, more preferably between 1 and 20 °.

3. Absorbent article (1) according to claim 1 or 2, wherein the angle beta has a value between 1 and 50°, more preferably between 2 and 40°.

4. Absorbent article (1) according to any of the previous claims, whereby the upper edge (22) of the front (12, 13) and/or the rear (11, 14) side panel displays a point symmetry with respect to a symmetry point located on the upper edge (22), centrally between the proximal (29) and the distal (20) edge.

5. Absorbent article (1) according to any one of the previous claims, whereby the ratio of the lower distance (B) and the side panel width (A) comprises a value between 0 and 1, whereby the lower distance (B) is defined as the difference in distance as measured along the longitudinal axis (L) between the point where the lower edge (21) crosses the proximal edge (29) and the point where the lower edge (21) crosses the distal edge (20), and the side panel width (A) is defined as the width of the side panel measured along the transverse axis (T) between the proximal (29) and the distal (20) edge.

6. Absorbent article (1) according to any of the previous claims, whereby the lower edge (21) of the front (12, 13) and/or the rear (11, 14) side panel displays a point symmetry with respect to a symmetry point located on the lower edge (21), centrally between the proximal (29) and the distal (20) edge.

7. Absorbent article (1) according to any of the previous claims, whereby the front (12, 13) and/or the rear (11, 14) side panel have a substantially trapezoidal shape.

8. Absorbent article (1) according to any of the previous claims, wherein the front (12, 13) and/or the rear (11, 14) side panel are formed from or comprise a non-woven material.

9. Absorbent article (1) according to any of the previous claims, whereby the absorbent article (1) comprises two front side panels (12, 13), one on each transversely opposed side edge of the front region (4), and two rear side panels (11, 14), one on each transversely opposed side edge of the rear region (2).

10. Absorbent article (1) according to claim 9, whereby the rear side panels (11, 14) comprise at least one fastening means (17, 18) attached to them which are able to connect to the front side panels (12, 13) in a refastenable manner, hereby joining together the front region (4) and the rear region (2).

11. Absorbent article (1) according to claim 9, whereby each front side panel (12, 13) and rear side panel (11, 14) attached to the same side edge of the chassis (5), are attached to each other so as to form a pant-like absorbent article.

12. A side panel for use as a front (12, 13) and/or a rear (11, 14) side panel in an absorbent article (1) according to any of the previous claims, wherein the side panel comprises two substantially parallel opposed length sides (32, 33) and two width sides (38, 39) extending between the two length sides, whereby the two width sides (38, 39) makes a non-zero angle alpha and a non-zero angle beta with an axis perpendicular to the two length sides, **characterized in that** the angle beta is larger than the angle alpha.

13. Side panel according to claim 12, whereby at least one of the two width sides (38, 39) displays a point symmetry with respect to a symmetry point located on that width side, centrally between the two length sides (32, 33).

14. Method to produce a side panel according to claim 12 or 13 comprising the steps of:
a. providing a layer of a web material (34) which is essentially endless along an x-axis and has two substantially parallel opposed length sides;
b. providing cutting means; and
c. cutting said layer with said cutting means at two distinct places along the x-axis, hereby forming a side panel with two width sides (38, 39) extending between the two length sides (32, 33); wherein the two width sides (38, 39) makes a non-zero angle alpha and a non-zero angle beta with a y-axis, which y-axis is substantially perpendicular to said x-axis, **characterized in that** the angle beta is larger than the angle alpha.

15. Method according to claim 14, whereby the angle alpha has a value between 0.5 and 30 °, more preferably between 1 and 20 °.

16. Method according to claim 14 or 15, whereby step c) of claim 14 is repeated to produce an essentially endless line of side panels adjacent to each other; whereby after being cut, each every other side panel in said line is translated along the y-axis and the remaining side panels are rotated over 180 ° along a z-axis, which z-axis is perpendicular to the x-axis and y-axis, and subsequently translated along the y-axis.

17. Method according to claim any of the previous claims 14-16, whereby no web material (34) is lost during cutting.

18. Method according to any of the previous claims 14-17, whereby at least one of the two width sides (38, 39) displays a point symmetry with respect to a symmetry point located on that width side, centrally between the two length sides (32, 33).

19. Method according to any of the previous claims 14-18, whereby the side panel has a substantially trapezoidal shape.

## Patentansprüche

1. Absorbierender Wegwerf-Inkontinenzartikel (1), der aufweist: eine flüssigkeitsdurchlässige oberste Lage (24), eine flüssigkeitsundurchlässige Unterlage (23) und einen absorbierenden Kern (15), der zwischen der obersten Lage (24) und der Unterlage (23) positioniert ist, wobei die oberste Lage (24), die Unterlage (23) und der absorbierende Kern (15) einen Körper (5) des absorbierenden Artikels (1) bilden; wobei der absorbierende Artikel (1) ferner aufweist: einen vorderen Bereich (4), einen hinteren Bereich (2) und einen Schrittbereich (3), wobei der Schrittbereich (3) den vorderen (4) und den hinteren (2) Bereich verbindet; eine Längsachse (L), die sich von dem vorderen Bereich (4) über den Schrittbereich (3) zu dem hinteren (2) Bereich erstreckt, und eine Querachse (T), die im Allgemeinen senkrecht zu der Längsachse (L) ist; wobei der Körper (5) zwei quer gegenüberliegende Seitenränder (6, 7), die sich im Wesentlichen entlang der Längsachse (L) erstrecken, und einen vorderen Rand (8) und einen hinteren Rand (9) jeweils an dem vorderen Bereich (4) und dem hinteren Bereich (2) aufweist, wobei der vordere Rand (8) und der hintere Rand (9) sich im Wesentlichen entlang der Querachse (T) erstrecken; wobei der absorbierende Artikel (1) ferner wenigstens ein vorderes Seitenelement (12, 13), das an dem vorderen Bereich (4) befestigt ist, und/oder wenigstens ein hinteres Seitenelement (11, 14), das an dem hinteren Bereich (2) befestigt ist, aufweist, wobei das vordere (12, 13) und/oder hintere (11, 14) Seitenelement einen proximalen Rand (29) und einen distalen Rand (20), die quer gegenüberliegend sind, und einen oberen Rand (22) und einen unteren Rand (21) aufweist, von denen sich jeder zwischen dem proximalen (29) zu dem distalen (20) Rand erstreckt, wobei der obere Rand (22) jeweils für ein vorderes Seitenelement (12, 13) und ein hinteres Seitenelement (11, 14) näher an dem vorderen Rand (8) oder dem hinteren Rand (9) des Körpers (5) als der untere Rand (21) angeordnet ist; wobei der proximale (29) und der distale (20) Rand im Wesentlichen parallel zueinander entlang der Längsachse (L) verlaufen, und wobei der obere Rand (22) des vorderen (12, 13) und/oder des hinteren (11, 14) Seitenelements einen Winkel Alpha ungleich null mit der Querachse (T) bilden, wobei der untere Rand (21) des vorderen (12, 13) und/oder des hinteren (11, 14) Seitenelements einen Winkel Beta mit der Querachse (T) bilden, **dadurch gekennzeichnet, dass** der Winkel Beta größer als der Winkel Alpha ist.

2. Absorbierender Artikel (1) nach Anspruch 1, wobei der Winkel Alpha einen Wert zwischen 0,5 und 30°, bevorzugter zwischen 1 und 20°, hat.

3. Absorbierender Artikel (1) nach Anspruch 1 oder 2, wobei der Winkel Beta einen Wert zwischen 1 und 50°, bevorzugter zwischen 2 und 40°, hat.

4. Absorbierender Artikel (1) nach einem der vorhergehenden Ansprüche, wobei der obere Rand (22) des vorderen (12, 13) und/oder des hinteren (11, 14) Seitenelements eine Punktsymmetrie in Bezug auf einen Symmetriepunkt zeigt, der auf dem oberen Rand (22) mittig zwischen dem proximalen (29) und dem distalen (20) Rand angeordnet ist.

5. Absorbierender Artikel (1) nach einem der vorhergehenden Ansprüche, wobei das Verhältnis des unteren Abstands (B) und der Seitenelementbreite (A) einen Wert zwischen 0 und 1 aufweist, wobei der untere Abstand (B) als die Differenz des Abstands entlang der Längsachse (L) zwischen dem Punkt, wo der untere Rand (21) den proximalen Rand (29) schneidet, und dem Punkt, wo der untere Rand (21) den distalen Rand (20) schneidet, gemessen definiert ist, und die Seitenelement Breite (A) als die Breite des Seitenelements entlang der Querachse (T) zwischen dem proximalen (29) und dem distalen (20) Rand gemessen definiert ist.

6. Absorbierender Artikel (1) nach einem der vorhergehenden Ansprüche, wobei der untere Rand (21) des vorderen (12, 13) und/oder des hinteren (11, 14) Seitenelements eine Punktsymmetrie in Bezug auf einen Symmetriepunkt zeigt, der auf dem unteren Rand (21) mittig zwischen dem proximalen (29) und dem distalen (20) Rand angeordnet ist.

7. Absorbierender Artikel (1) nach einem der vorhergehenden Ansprüche, wobei das vordere (12, 13) und/oder das hintere (11, 14) Seitenelement eine im Wesentlichen trapezförmige Form haben.

8. Absorbierender Artikel (1) nach einem der vorhergehenden Ansprüche, wobei das vordere (12, 13) und/oder das hintere (11, 14) Seitenelement aus einem Vliesmaterial ausgebildet sind oder dieses aufweisen.

9. Absorbierender Artikel (1) nach einem der vorhergehenden Ansprüche, wobei der absorbierende Artikel (1) zwei vordere Seitenelemente (12, 13), eines auf jedem quer gegenüberliegenden Seitenrand des vorderen Bereichs (4), und zwei hintere Seitenelemente (11, 14), eines auf jedem quer gegenüberliegenden Seitenrand des hinteren Bereichs (2), aufweist.

10. Absorbierender Artikel (1) nach Anspruch 9, wobei die hinteren Seitenelemente (11, 14) wenigstens eine an ihnen befestigte Verschlusseinrichtung (17, 18) aufweisen, die fähig ist, in einer wiederverschließbaren Weise mit den vorderen Seitenelementen (12, 13) zu verbinden, wodurch der vordere Bereich (4) und der hintere Bereich (2) miteinander verbunden werden.

11. Absorbierender Artikel (1) nach Anspruch 9, wobei jedes vordere Seitenelement (12, 13) und hintere Seitenelement (11, 14), die an dem gleichen Seitenrand des Körpers (5) befestigt sind, aneinander befestigt sind, um einen höschenartigen absorbierenden Artikel zu bilden.

12. Seitenelement für die Verwendung als ein vorderes (12, 13) und/oder ein hinteres (11, 14) Seitenelement in einem absorbierenden Artikel (1) nach einem der vorhergehenden Ansprüche, wobei das Seitenelement zwei im Wesentlichen parallele gegenüberliegenden Längsseiten (32, 33) und zwei Breitenseiten (38, 39), die sich zwischen den Längsseiten erstrecken, aufweist, wobei die zwei Breitenseiten (38, 39) einen Winkel Alpha ungleich null und einen Winkel Beta ungleich null mit einer Achse senkrecht zu den zwei Längsseiten bilden, **dadurch gekennzeichnet, dass** der Winkel Beta größer als der Winkel Alpha ist.

13. Seitenelement nach Anspruch 12, wobei wenigstens eine der zwei Breitenseiten (38, 39) eine Punktsymmetrie in Bezug auf einen Symmetriepunkt zeigt, der auf dieser Breitenseite mittig zwischen den zwei Längsseiten (23, 33) angeordnet ist.

14. Verfahren zur Herstellung eines Seitenelements nach Anspruch 12 oder 13, das die folgenden Schritte aufweist:
a. Bereitstellen einer Schicht aus einem Bahnmaterial (34), das entlang einer x-Achse im Wesentlichen endlos ist und zwei im Wesentlichen parallele gegenüberliegende Längsseiten hat;
b. Bereitstellen von Schneideinrichtungen; und
c. Schneiden der Schicht mit den Schneideinrichtungen an zwei unterschiedlichen Orten entlang der x-Achse, wodurch ein Seitenelement mit zwei Breitenseiten (38, 39), das sich zwischen den zwei Längsseiten (32, 33) erstreckt, ausgebildet wird; wobei die zwei Breitenseiten (38, 39) einen Winkel Alpha ungleich null und einen Winkel Beta ungleich null mit einer y-Achse bilden, wobei die y-Achse im Wesentlichen senkrecht zu der x-Achse ist, **dadurch gekennzeichnet, dass** der Winkel Beta größer als der Winkel Alpha ist.

15. Verfahren nach Anspruch 14, wobei der Winkel Alpha einen Wert zwischen 0,5 und 30°, bevorzugter zwischen 1 und 20°, hat.

16. Verfahren nach Anspruch 14 oder 15, wobei der Schritt c) von Anspruch 14 widerholt wird, um eine im Wesentlichen endlose Reihe von zueinander benachbarten Seitenelementen herzustellen; wobei nach dem Schneiden jedes zweite Seitenelement in der Reihe entlang der y-Achse verschoben wird und die verbleibenden Seitenelemente entlang der z-Achse um 180° gedreht werden, wobei die z-Achse senkrecht zu der x-Achse und der y-Achse ist, und anschließend entlang der y-Achse verschoben wird.

17. Verfahren nach einem der vorhergehenden Ansprüche 14 - 16, wobei während des Schneidens kein Bahnmaterial (34) verlorengeht.

18. Verfahren nach einem der vorhergehenden Ansprüche 14 - 17, wobei wenigstens eine der zwei Breitenseiten (38, 39) eine Punktsymmetrie in Bezug auf einen Symmetriepunkt zeigt, der auf dieser Breitenseite mittig zwischen den zwei Längsseiten (32, 33) angeordnet ist.

19. Verfahren nach einem der vorhergehenden Ansprüche 14 - 18, wobei das Seitenelement eine im Wesentlichen trapezförmige Form hat.

## Revendications

1. Article absorbant jetable pour l'incontinence (1) comprenant : une feuille supérieure perméable aux liquides (24), une feuille inférieure imperméable aux liquides (23) et un cœur absorbant (15) positionné entre la feuille supérieure (24) et la feuille inférieure(23), moyennant quoi la feuille supérieure (24), la feuille inférieure (23) et le cœur absorbant (15) forment un châssis (5) de l'article absorbant (1) ; l'article absorbant (1) comprenant en outre une région avant (4), une région arrière (2) et une région d'entrejambe (3), ladite région d'entrejambe (3) reliant les régions avant (4) et arrière (2) ; un axe longitudinal (L) qui s'étend à partir de la région avant (4) sur la région d'entrejambe (3) jusqu'à la région arrière (2), et un axe transversal (T) qui est généralement perpendiculaire à l'axe longitudinal (L) ; moyennant quoi le châssis (5) comprend deux côtés latéraux transversalement opposés (6, 7) s'étendant sensiblement le long de l'axe longitudinal (L), et un bord avant (8) et un bord arrière (9) respectivement au niveau de la région avant (4) et de la région arrière (2), le bord avant (8) et le bord arrière (9) s'étendant sensiblement le long de l'axe transversal (T) ; l'article absorbant (1) comprenant en outre au moins un panneau latéral avant (12, 13) fixé à la région avant (4) et/ou au moins un panneau latéral arrière (11, 14) fixé à la région arrière (2), moyennant quoi lesdits panneaux latéraux avant (12, 13) et/ou arrière (11, 14) comprennent un bord proximal (29) et un bord distal (20) qui sont transversalement opposés, et un bord supérieur (22) et un bord inférieur (21), s'étendant chacun entre les bords proximal (29) et distal (20), le bord supérieur (22) étant situé plus proche du bord avant (8) ou du bord arrière (9) du châssis (5) que le bord inférieur (21) respectivement pour un panneau latéral avant (12, 13) et un panneau latéral arrière (11, 14) ; dans lequel les bords proximal (29) et distal (20) s'étendent sensiblement parallèles l'un à l'autre le long de l'axe longitudinal (L) et moyennant quoi le bord supérieur (22) des panneaux latéraux avant (12, 13) et/ou arrière (11, 14) forme un angle alpha non nul avec l'axe transversal (T), moyennant quoi le bord inférieur (21) des panneaux latéraux avant (12, 13) et/ou arrière (11, 14) forme un angle bêta avec l'axe transversal (T), **caractérisé en ce que** l'angle bêta est supérieur à l'angle alpha.

2. Article absorbant (1) selon la revendication 1, moyennant quoi l'angle alpha a une valeur entre 0,5 et 30°, de manière davantage préférée entre 1 et 20°.

3. Article absorbant (1) selon la revendication 1 ou 2, dans lequel l'angle bêta a une valeur entre 1 et 50°, de manière davantage préférée entre 2 et 40°.

4. Article absorbant (1) selon l'une quelconque des revendications précédentes, moyennant quoi le bord supérieur (22) des panneaux latéraux avant (12, 13) et/ou arrière (11, 14) affiche une symétrie ponctuelle par rapport à un point de symétrie situé sur le bord supérieur (22), centralement entre les bords proximal (29) et distal (20).

5. Article absorbant (1) selon l'une quelconque des revendications précédentes, moyennant quoi le rapport de la distance inférieure (B) et de la largeur de panneau latéral (A) comprend une valeur entre 0 et 1, moyennant quoi la distance inférieure (B) est définie comme la différence de distance mesurée le long de l'axe longitudinal (L) entre le point où le bord inférieur (21) croise le bord proximal (29) et le point où le bord inférieur (21) croise le bord distal (20), et la largeur de panneau latéral (A) est définie comme la largueur du panneau latéral mesurée le long de l'axe transversal (T) entre les bords proximal (29) et distal (20).

6. Article absorbant (1) selon l'une quelconque des revendications précédentes, moyennant quoi le bord inférieur (21) des panneaux latéraux avant (12, 13) et/ou arrière (11, 14) affiche une symétrie ponctuelle par rapport à un point de symétrie situé sur le bord inférieur (21), centralement entre les bords proximal (29) et distal (20).

7. Article absorbant (1) selon l'une quelconque des revendications précédentes, moyennant quoi les panneaux latéraux avant (12, 13) et/ou arrière (11, 14) ont une forme sensiblement trapézoïdale.

8. Article absorbant (1) selon l'une quelconque des revendications précédentes, dans lequel les panneaux latéraux avant (12, 13) et/ou arrière (11, 14) sont formés à partir d'un, ou comprennent un, matériau non tissé.

9. Article absorbant (1) selon l'une quelconque des revendications précédentes, moyennant quoi l'article absorbant (1) comprend deux panneaux latéraux avant (12, 13), un sur chaque bord latéral transversalement opposé de la région avant (4), et deux panneaux latéraux arrière (11, 14), un sur chaque bord latéral transversalement opposé de la région arrière (2).

10. Article absorbant (1) selon la revendication 9, moyennant quoi les panneaux latéraux arrière (11, 14) comprennent au moins un moyen de fixation (17, 18) fixé à eux qui est capable de se relier aux panneaux latéraux avant (12, 13) de manière refixable, joignant ainsi ensemble la région avant (4) et la région arrière (2).

11. Article absorbant (1) selon la revendication 9, moyennant quoi chaque panneau latéral avant (12, 13) et panneau latéral arrière (11, 14) fixés au même bord latéral du châssis (5), sont fixés l'un à l'autre pour former un article absorbant de type culotte.

12. Panneau latéral pour son utilisation en tant que panneaux latéraux avant (12, 13) et/ou arrière (11, 14) dans un article absorbant (1) selon l'une quelconque des revendications précédentes, dans lequel le panneau latéral comprend deux côtés de longueur opposés sensiblement parallèles (32, 33) et deux côtés de largeur (38, 39) s'étendant entre les deux côtés de longueur, moyennant quoi les deux côtés de largeur (38, 39) forment un angle alpha non nul avec un axe perpendiculaire aux deux côtés de longueur, **caractérisé en ce que** l'angle bêta est supérieur à l'angle alpha.

13. Panneau latéral selon la revendication 12, moyennant quoi au moins l'un des deux côtés de largeur (38, 39) affiche une symétrie ponctuelle par rapport à un point de symétrie situé sur ce côté de largeur, centralement entre les deux côtés de longueur (32, 33).

14. Procédé de production d'un panneau latéral selon la revendication 12 ou 13 comprenant les étapes de :
a. fourniture d'une couche d'un matériau de nappe (34) qui est sensiblement sans fin le long d'un axe x et a deux côtés de longueur opposés sensiblement parallèles ;
b. fourniture d'un moyen de découpe ; et
c. découpe de ladite couche avec ledit moyen de découpe au niveau de deux emplacements distincts le long de l'axe x, formant ainsi un panneau latéral avec deux côtés de largeur (38, 39) s'étendant entre les deux côtés de longueur (32, 33) ; dans lequel les deux côtés de largeur (38, 39) forment un angle alpha non nul et un angle bêta non nul avec un axe y, l'axe y étant sensiblement perpendiculaire au dit axe x, **caractérisé en ce que** l'angle bêta est supérieur à l'angle alpha.

15. Procédé selon la revendication 14, moyennant quoi l'angle alpha a une valeur entre 0,5 et 30°, de manière davantage préférée entre 1 et 20°.

16. Procédé selon la revendication 14 ou 15, moyennant quoi l'étape c) de la revendication 14 est répétée pour produire une ligne sensiblement sans fin de panneaux latéraux adjacents les uns aux autres ; moyennant quoi après avoir été découpé, un panneau latéral sur deux dans ladite ligne est translaté le long de l'axe y et les panneaux latéraux restants sont pivotés sur 180° le long d'un axe z, l'axe z étant perpendiculaire à l'axe x et à l'axe y, et ensuite translatés le long de l'axe y.

17. Procédé selon l'une quelconque des revendications précédentes 14 à 16, moyennant quoi aucun matériau de bande (34) n'est perdu durant la découpe.

18. Procédé selon l'une quelconque des revendications précédentes 14 à 17, moyennant quoi au moins l'un des deux côtés de largeur (38, 39) affiche une symétrie ponctuelle par rapport à un point de symétrie situé sur ce côté de largeur, centralement entre les deux côtés de longueur (32, 33).

19. Procédé selon l'une quelconque des revendications précédentes 14 à 18, moyennant quoi le panneau latéral a une forme sensiblement trapézoïdale.
